# EUROPEAN PATENT APPLICATION

(11) **EP 4 442 704 A1**
(43) Date of publication of application: **09.10.2024**
(21) Application number: 22901376.8
(22) Date of filing: 30.11.2022
(51) Int. Cl.: C07K 16/00, C40B 40/10

(54) **PEPTIDE HAVING FRAMEWORK SEQUENCE FOR RANDOM REGION PLACEMENT AND PEPTIDE LIBRARY COMPOSED OF SAID PEPTIDE**

(30) Priority: 01.12.2021 JP 2021195792
(71) Applicant: Epsilon Molecular Engineering Inc., Saitama City, Saitama 338-8570 (JP); Mitsui Knowledge Industry Co., Ltd., Tokyo 105-6215 (JP)
(72) Inventor: MURAKAMI Taihei, Saitama City, Saitama 338-8570 (JP); KUMACHI Shigefumi, Saitama City, Saitama 338-8570 (JP); TSUCHIYA Masayuki, Saitama City, Saitama 338-8570 (JP); HIRAYAMA Hiroyuki, Yokohama City, Kanagawa 244-0003 (JP)
(74) Representative: Kuhnen & Wacker Patent- und Rechtsanwaltsbüro PartG mbB
(86) International application number: PCT/JP2022/044235
(87) International publication number: WO 2023/100944

(57) **Abstract**

Provided are a peptide consisting of three complementarity determining regions and four framework regions, in which a framework 1 comprises an amino acid sequence represented by SEQ ID NO: 1, a framework 2 comprises an amino acid sequence represented by SEQ ID NO: 2, a framework 3 comprises an amino acid sequence represented by SEQ ID NO: 7, a framework 4 comprises an amino acid sequence represented by SEQ ID NO: 32, a complementarity determining region 1 is an amino acid sequence consisting of 10 amino acids, a complementarity determining region 2 is an amino acid sequence consisting of 16 or 17 amino acids, and a complementarity determining region 3 is an amino acid sequence consisting of 6, 12 or 15 amino acids; and a peptide library including the peptide.

## Description

### Technical Field

The present invention relates to a peptide having a framework sequence for random region placement and a peptide library composed of the peptide.

### Background Art

Conventionally, it has been known that a living organism has a biological defense mechanism that recognizes self versus non-self, and the biological defense mechanism involves cellular immunity and humoral immunity. Of these, the humoral immunity is such that an antibody produced by B cells recognizes a foreign substance entering the body, and binds specifically to an antigen (target) present on the surface of the foreign substance, followed by removal of the foreign substance from the living organism.

The antibody is a protein called "immunoglobulin" (which may be referred to as "Ig" hereinafter), which is classified into IgA, IgD, IgE, IgG and IgM. Among them, IgG is called a common antibody because it is produced in large amounts, and consists of a heavy chain and a light chain in many mammals. However, in camels, alpacas and other camelids or sharks and other cartilage fish, IgG consists of only a heavy chain. Such an antibody consisting of a single domain having only a heavy chain is called a heavy chain antibody. The antibody can bind to an antigen only in a variable region, and thus is also called a single domain antibody, Nanobody (registered trademark), or VHH.

The VHH, whose amino acid sequences are precisely defined, can be used as a tool for biochemical analysis by an immunoprecipitation method or the like or as a tool for use in immunohistochemistry like conventional antibodies such as IgG.

The VHH is known to differ from the conventional antibodies in binding site to an antigen. Specifically, it is known that the conventional antibody binds to an epitope on a convex part of the antigen, whereas the VHH binds to an epitope of an antigen which forms a gap, a cleft or the like of an antigen (see Prior Art Literatures 1 to 3).

Thus, development of therapeutic agents that are difficult to develop using conventional antibodies requires a search for candidate proteins having the binding characteristics of VHH obtained from a camelid.

The above-described search typically comprises screening a peptide library using a display method such as a phage display method (see Prior Art Literature 1). Here, the peptide library can be defined as a "large collection of peptides having a systematic combination of amino acids". The peptides include naive peptides obtained from various animals or the like, semisynthetic peptides obtained by modification of the naive peptides, and synthetic peptides obtained by chemical synthesis.

It is known that epitope peptide mapping, preparation of a peptide library for development of vaccine, and the like can be performed by screening the above-described synthetic peptide library.

### Citation List

### Patent Literature

Patent Literature 1: International Publication No. WO 2003/025020

### Non Patent Literature

Non Patent Literature 1: Marc Lauwereys, et al., EMBO J 17, (13): 3512, 1998
Non Patent Literature 2: M. Arbabi Ghahroudi, et al., FEBS Letters 414 (1997): 521-526

### Summary of Invention

### Technical Problem

In order to obtain diverse candidate peptides through a search, a peptide library with large diversity is required. Conventional art 1 (mRNA display method) is an excellent technique in terms of a library size. However, the mRNA display method has a problem in that an intended peptide cannot be obtained with stability because mRNA is likely to undergo degradation by RNase in an aqueous solution.

Conventional art 2 (phage display method) is an excellent technique in terms of stability of display. However, for acquiring a large amount of a peptide displayed on a phage, it is necessary that a construct which expresses DNA encoding the displayed peptide be prepared, and finally introduced into *Escherichia coli* or other bacteria and expressed, and thus a translation system using cells is required.

Therefore, there's an issue with the limited size of usable libraries, as well as the inability to display a peptide if its expression proves harmful or fatal to the host.

In addition, there is a problem in that although a tertiary structure of a protein or peptide can be predicted from a secondary structure to some extent, it is not possible to predict a binding mode to a target peptide, to design such a structure to serve as a binding site, and then to express it as predicted.

Thus, there is a strong social demand for a framework having a sequence on which a CDR with a desired length can be placed. In particular, there is a strong social demand for a framework sequence which can be used to efficiently screen single domain antibodies such as VHH obtained from camelids, and enables obtaining, through the screening, a peptide having structural characteristics consistent with the design.

Further, there is a strong social demand for a peptide library composed of peptides having the framework described above.

### Solution to Problem

Under these circumstances, the inventors of the present invention have extensively conducted studies to complete the invention of the present application.

That is, a first aspect of the present invention is a peptide consisting of three complementarity determining regions and four framework regions (which may be abbreviated as "FR" hereinafter), and comprising a sequence represented by SEQ ID NO: 1 as a framework 1 (hereinafter, referred to as "FR1").

[SEQ ID NO: 1] EVQLVESGGGLVQPGGSLRLSCAAS

The FR preferably comprises an amino acid sequence represented by SEQ ID NO: 2 as a framework 2 (hereinafter, referred to as "FR2"). Here, X in the sequence of SEQ ID NO: 2 represents an arbitrary amino acid, which is preferably any amino acid selected from the group consisting of tyrosine, valine, phenylalanine, leucin, arginine, glycine and tryptophane from the viewpoint of tertiary structure stability.

### [SEQ ID NO: 2] WXRQAPGKGXEXVA

The FR2 is more preferably any sequence selected from the group consisting of amino acid sequences represented by SEQ ID NOS: 3 to 6 from the viewpoint of tertiary structure stability.
[SEQ ID No. 3] WYRQAPGKGLEWVA
[SEQ ID No. 4] WVRQAPGKGLEWVA
[SEQ ID No. 5] WFRQAPGKGREFVA
[SEQ ID No. 6] WFRQAPGKGREGVA

The FR preferably comprises an amino acid sequence represented by SEQ ID NO: 7 as a framework 3 (hereinafter, referred to as "FR3"). Here, X in the sequence of SEQ ID NO: 7 represents an arbitrary amino acid, which is preferably any amino acid selected from the group consisting of leucine, valine, alanine (A), arginine (R) and lysine (K).

[SEQ ID NO: 7] RFTISRDNSKNTXYLQMNSLRAEDTAVYYCXX

The FR3 is preferably any sequence selected from the group consisting of amino acid sequences represented by SEQ ID NOS: 8 to 31 described below.
[SEQ ID No. 8] RFTISRDNAKNTLYLQMNSLRAEDTAVYYCNA
[SEQ ID No. 9] RFTISRDNAKNTLYLQMNSLRAEDTAVYYCNV
[SEQ ID No. 10] RFTISRDNAKNTLYLQMNSLRAEDTAVYYCNR
[SEQ ID No. 11] RFTISRDNAKNTLYLQMNSLRAEDTAVYYCNL
[SEQ ID No. 12] RFTISRDNAKNTVYLQMNSLRAEDTAVYYCNA
[SEQ ID No. 13] RFTISRDNAKNTVYLQMNSLRAEDTAVYYCNV
[SEQ ID No. 14] RFTISRDNAKNTVYLQMNSLRAEDTAVYYCNR
[SEQ ID No. 15] RFTISRDNAKNTVYLQMNSLRAEDTAVYYCNL
[SEQ ID No. 16] RFTISRDNAKNTLYLQMNSLRAEDTAVYYCAV
[SEQ ID No. 17] RFTISRDNAKNTLYLQMNSLRAEDTAVYYCAR
[SEQ ID No. 18] RFTISRDNAKNTLYLQMNSLRAEDTAVYYCAL
[SEQ ID No. 19] RFTISRDNAKNTVYLQMNSLRAEDTAVYYCAV
[SEQ ID No. 20] RFTISRDNAKNTVYLQMNSLRAEDTAVYYCAR
[SEQ ID No. 21] RFTISRDNAKNTVYLQMNSLRAEDTAVYYCAL
[SEQ ID No. 22] RFTISRDNAKNTLYLQMNSLRAEDTAVYYCKA
[SEQ ID No. 23] RFTISRDNAKNTLYLQMNSLRAEDTAVYYCKV
[SEQ ID No. 24] RFTISRDNAKNTLYLQMNSLRAEDTAVYYCKR
[SEQ ID No. 25] RFTISRDNAKNTLYLQMNSLRAEDTAVYYCKL
[SEQ ID No. 26] RFTISRDNAKNTVYLQMNSLRAEDTAVYYCKA
[SEQ ID No. 27] RFTISRDNAKNTVYLQMNSLRAEDTAVYYCKV
[SEQ ID No. 28] RFTISRDNAKNTVYLQMNSLRAEDTAVYYCKR
[SEQ ID No. 29] RFTISRDNAKNTVYLQMNSLRAEDTAVYYCKL
[SEQ ID No. 30] RFTISRDNAKNTLYLQMNSLRAEDTAVYYCAA
[SEQ ID No. 31] RFTISRDNAKNTVYLQMNSLRAEDTAVYYCAA

The FR preferably comprises an amino acid sequence represented by SEQ ID NO: 32 in the following sequence listing as a framework 4 (hereinafter, referred to as "FR4").

[SEQ ID NO: 32] WGQGTLVTVSS

The FR1 to FR4 preferably consist, respectively, of 22 to 26 amino acids, 12 to 16 amino acids, 29 to 33 amino acids and 9 to 13 amino acids. The FR1 to FR4 more preferably consist, respectively, of 25 amino acids, 14 amino acids, 32 amino acids and 11 amino acids.

The peptide preferably comprises a complementarity determining region 1 (hereinafter, referred to as "CDR1") consisting of 8 to 12 amino acids, a complementarity determining region 2 (hereinafter, referred to as "CDR2") consisting of 14 to 18 amino acids, and a complementarity determining region 3 (CDR3) consisting of any amino acid sequence selected from the group consisting of sequences of 6, 12 and 15 amino acids. The peptide more preferably comprises CDR1 consisting of 10 amino acids, CDR2 consisting of 16 or 17 amino acids, and CDR3 consisting of 6, 12 or 15 amino acids.

A second aspect of the present invention is a peptide library composed of the peptides. A preferred peptide library is a peptide library in which a peptide constituting the peptide library consists of three complementarity determining regions and four framework regions,
a framework 1 comprises an amino acid sequence represented by SEQ ID NO: 1,
a framework 2 comprises an amino acid sequence represented by SEQ ID NO: 2,
a framework 3 comprises an amino acid sequence represented by SEQ ID NO: 7,
a framework 4 comprises an amino acid sequence represented by SEQ ID NO: 32,
a complementarity determining region 1 is an amino acid sequence consisting of 10 amino acids,
a complementarity determining region 2 is an amino acid sequence consisting of 16 or 17 amino acids, and
a complementarity determining region 3 is an amino acid sequence consisting of 6, 12 or 15 amino acids.

A particularly preferred peptide library is a peptide library in which a peptide constituting the peptide library consists of three complementarity determining regions and four framework regions,
a framework 1 comprises an amino acid sequence represented by SEQ ID NO: 1,
a framework 2 comprises an amino acid sequence represented by any of SEQ ID NOS: 3 to 6,
a framework 3 comprises an amino acid sequence represented by any of SEQ ID NOS: 8 to 31,
a framework 4 comprises an amino acid sequence represented by SEQ ID NO: 32,
a complementarity determining region 1 is an amino acid sequence consisting of 10 amino acids,
a complementarity determining region 2 is an amino acid sequence consisting of 16 or 17 amino acids, and
a complementarity determining region 3 is an amino acid sequence consisting of 6, 12 or 15 amino acids.

The library size of the peptide library is preferably about 10¹² to about 10¹⁴. The library is preferably a single domain antibody derived from the heavy chain antibody as described above, or a polyvalent form in which the heavy chain antibodies are bound to one another.

The polyvalent form is preferably any one selected from the group consisting of di- to pentamers in which the antibodies are bound to one another via a linker.

### Advantageous Effects of Invention

According to the present invention, it is possible to obtain a framework sequence having a sequence on which CDR with a desired length can be placed, and a peptide comprising the framework sequence. Furthermore, it is possible to obtain a framework sequence which can be used to efficiently screen single domain antibodies, and enables the acquisition of a peptide having structural characteristics as designed. Moreover, it is possible to construct a peptide library composed of the above-described peptides.

### Brief Description of Drawings

[Figure 1] Figure 1 is a schematic diagram showing classification of peptides into three groups on the basis of the structure of a CDR3 region of the peptide. Figure 1(A) shows an Upright type, Figure 1(B) shows a Half-roll type, and Figure 1(C) shows a Roll type. In the figure, L1 to L3 denote CDR3 regions in respective molecules.
[Figure 2] Figure 2 is a schematic diagram showing the distance between Cα atoms (which may be referred to as a "Cα distance" hereinafter) of the peptide and the pseudo-dihedral angle between Cα atoms (which may be referred to as a "dihedral angle" hereinafter), which were used as criteria for the classification in Figure 1.
[Figure 3] Figure 3 shows charts obtained by plotting a Cα distance and a dihedral angle of VHH. Figure 3(A) shows the distributions of the Cα distance and the dihedral angle for VHH classified into Upright type, Half-roll type and Roll type.
Figure 3(B) shows the Cα distances and the dihedral angles of VHH and human VH in the same manner as described above.

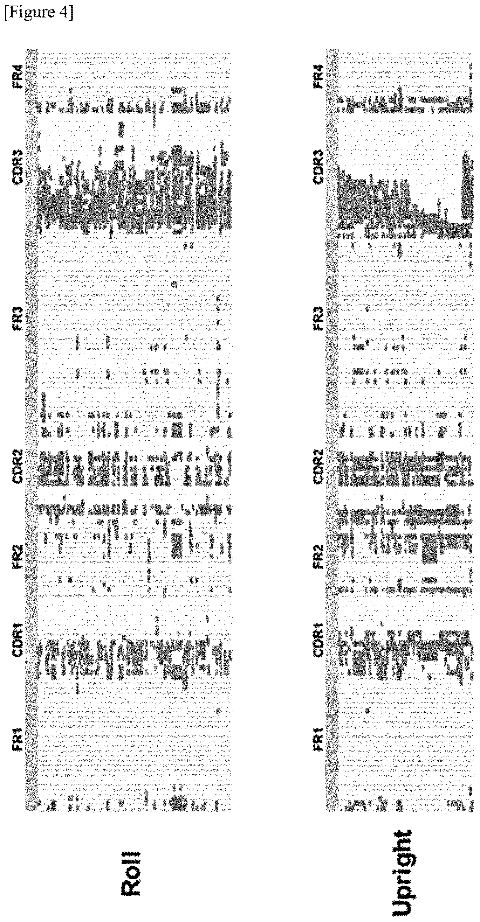

[Figure 4] Figure 4 is a diagram of comparison between amino acid sequences in the upright type and the roll type as shown in Figure 1.
[Figure 5] Figure 5 is a graph showing the distribution of the CDR3 length of VHH and human VH for above-described type of VHH.
[Figure 6] Figure 6 shows designs of framework regions in a VHH sub-library (upright type or roll type). The alphabet shown below each amino acid sequence indicates a mutated amino acid and its position.
[Figure 7] Figure 7 shows a procedure for preparing a DNA sequence of full-length VHH.

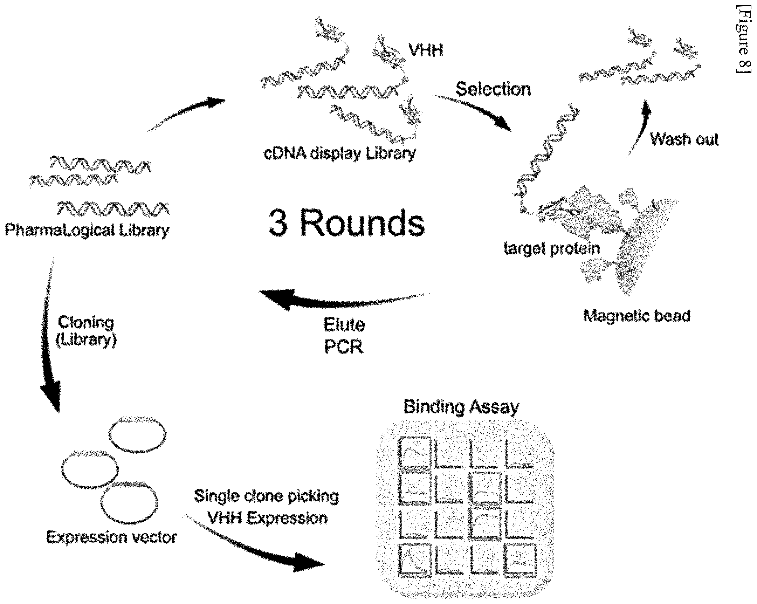

[Figure 8] Figure 8 schematically shows a flow of screening when "The Month" platform is used.
[Figure 9] Figure 9 shows the characteristics of anti-GPC3-VHH obtained through screening.
[Figure 10] Figure 10 shows the characteristics of VHH obtained through screening.
Figure 10(A) is a graph obtained by plotting a relationship between heat stability and a dissociation constant of the obtained VHH. Figure 10(C) shows the results of actually measuring the dissociation constant. Figure 10(B) is a graph showing a relationship between a thermal denaturation midpoint (Tm value) and an aggregation initiation temperature (Tagg value).
[Figure 11] Figure 11 shows the results of the flow cytometric analysis which evaluated the ability of VHH to bind to a target protein expressed on cell surfaces.

### Description of Embodiment

The present invention will be described below with reference to the appended drawings as necessary. It should be understood that terms used herein are used in the ordinary sense in the art unless otherwise specified. Therefore, unless otherwise defined, all technical terms and scientific and technological terms used herein have the same meanings as those that are commonly understood by a person skilled in the art to which the present invention pertains. Herein, terms in a singular form can include plural forms unless otherwise specified.

The sequences used in the present invention are as shown in Table 1.

The present invention is, as described above, a peptide (a) consisting of three CDRs and four FRs and (b) comprising specific sequences described below as FR1, FR2, FR3 and FR4.

### (1) Peptide constituting FR1 to FR4

A peptide constituting the FR1 to FR4 can be designed and prepared as follows. First, the amino acid sequences of the human framework sequence IGHV3-23*01 DP-47 (FR1 to FR4 are shown as SEQ ID NOS: 34 to 37 in Table 1) are selected as basic sequences for use in design of framework sequence in the present invention for the reason of antigenicity and homology with VHH. FR to be changed in design is determined while the shape of a peptide that is ultimately formed is taken into account.

Here, it is preferable that the FR1 to FR4 consist, respectively, of 22 to 26 amino acids, 12 to 16 amino acids, 29 to 33 amino acids and 9 to 13 amino acids from the viewpoint of tertiary structure stability. It is more preferable that the FR1 to FR4 consist, respectively, of 24 amino acids, 14 amino acids, 32 amino acids and 11 amino acids from the viewpoint of tertiary structure stability.

For example, if it may be desirable to fix the sequence of an N-terminal region and a C-terminal region of a peptide for maintaining the stability of the steric structure of the peptide, the sequences of FR2 and FR3 may be changed with no change made to FR1 and FR4. In this case, in the context of the steric structure of CDR3, as described above, amino acids at desired sites in FR2 and FR3 can be replaced with another amino acid.

Here, for the sequence of FR2, the amino acid at the C-terminus of FR2 of the human framework sequence IGHV3-23*01 DP-47 can be changed from serine (S) to alanine (A) from the viewpoint of the stability of the three-dimensional structure of CDR2. Amino acids at other positions can be replaced with another amino acid. For example, the 2nd, 10th or 12th amino acid from the N-terminus can be replaced with any amino acid selected from the group consisting of tyrosine (Y), valine (V), phenylalanine (F), leucine (L), arginine (R), glycine (G) and tryptophane (W).

In this case, FR2 containing, at the above-described site, an amino acid to be replaced can be presented as the following amino acid sequence represented by SEQ ID NO: 2.
[SEQ ID NO: 2] WXRQAPGKGXEXVA

Here, X at the 2nd position from the N-terminus (hereinafter, referred to "X2") may be any amino acid selected from the group consisting of tyrosine, valine (V) and phenylalanine (F), X at the 10th position from the N-terminus (X10) may be any amino acid selected from the group consisting of arginine (R) or leucine (L), and X at the 12th position from the N-terminus (X12) may be any amino acid selected from the group consisting of tryptophane (W), phenylalanine (F) and glycine (G).

For example, the FR2 preferably has a sequence represented by any amino acid sequence selected from the group consisting of sequences represented by SEQ ID NOS: 3 to 6. This is because by performing the replacement in this way, the steric structure of the peptide can be controlled to some extent.

**[Table 1]**

| Sequence | SEQ ID NO | Region |
|---|---|---|
| EVQLVESGGGLVQPGGSLRLSCAAS | 1 | FR1 |
| WXRQAPGKGXEXVA | 2 | FR2 |
| WYRQAPGKGLEWVA | 3 | FR2 (Upright type) |
| WVRQAPGKGLEWVA | 4 | FR2 (Upright type) |
| WFRQAPGKGREFVA | 5 | FR2 (Roll type) |
| WFRQAPGKGREGVA | 6 | FR2 (Roll type) |
| RFTISRDNAKNTXYLQMNSLRAEDTAVYYCXX | 7 | FR3 (Upright type) |
| RFTISRDNAKNTLYLQMNSLRAEDTAVYYCNA | 8 | PR3 (Upright type) |
| RFTISRDNAKNTLYLQMNSLRAEDTAVYYCNV | 9 | FR3 (Upright type) |
| RFTISRDNAKNTLYLQMNSLRAEDTAVYYCNR | 10 | FR3 (Upright type) |
| RFTISRDNAKNTLYLQMNSLRAEDTAVYYCNL | 11 | FR3 (Upright type) |
| RFTISKDNAKNTVYLQMNSLRAEDTAVYYCM | 12 | FR3 (Upright type) |
| RFTISRDNAKNTVYLQMNSLRAEDTAVYYCNV | 13 | FR3 (Upright type) |
| RFTISRDNAKNTVYLQMNSLRAEDTAVYYCNR | 14 | FR3 (Upright type) |
| RFTISRDNAKNTVYLQMNSLRAEDTAVYYCNL | 15 | FR3 (Upright type) |
| RFTISRDNAKNTLYLQMNSLRAEDTAVYYCAV | 16 | FR3 (Upright type) |
| RFTISRDNAKNTLYLQMNSLRAEDTAVYYCAR | 17 | FR3 (Upright type) |
| RFTISRDNAKNTLYLQMNSLRAEDTAVYYCAL | 18 | FR3 (Upright type) |
| RFTISRDNAKNTVYLQMNSLRAEDTAVYYCAV | 19 | FR3 (Upright type) |
| RFTISRDNAKNTVYLQMNSLRAEDTAVYYCAR | 20 | FR3 (Upright type) |
| RFTISKDNAKNTVYLQMNSLRAEDTAVYYCAL | 21 | FR3 (Upright type) |
| RFTISRDNAKNTLYLQMNSLRAEDTAVYYCKA | 22 | FR3 (Upright type) |
| RFTISRDNAKNTLYLQMNSLRAEDTAVYYCKV | 23 | FR3 (Upright type) |
| RFTISRDNAKNTLYLQMNSLRAEDTAVYYCKR | 24 | FR3 (Upright type) |
| RFTTSRDNAKNTLYLQMNSLRAEDTAVYYCKL | 25 | PR3 (Upright type) |
| RFTISRDNAKNTVYLQMNSLRAEDTAVYYCKA | 26 | FR3 (Upright type) |
| RFTISRDNAKNTVYLQMNSLRAEDTAVYYCKV | 27 | FR3 (Upright type) |
| RFTISRDNAKNTVYLQMNSLRAEDTAVYYCKR | 28 | FR3 (Upright type) |
| RFTISKDNAKNTVYLQMNSLRABDTAVYYCKL | 29 | FR3 (Upright type) |
| RFTISRDNAKNTLYLQMNSLRAEDTAVYYCAA | 30 | FR3 (Roll/Upright type) |
| RFTISRDNAKNTVYLQMNSLRAEDTAVYYCAA | 31 | FR3 (Roll/Upright type) |
| WGQGTLVTVSS | 32 | FR4 |
| XXXXXXXXXX | - | CDR1 |
| XXXXXXXXXXXXXXXX | - | CDR2 (Upright type) |
| XXXXXXXXXXXXXXXXX | - | CDR2 (Roll type) |
| XXXXXX | - | CDR3 (Upright type) |
| XXXXXXXXXXXX | - | CDR3 (Roll/Uprighttype) |
| XXXXXXXXXXXXXXX | - | CDR3 (Roll type) |
| AANAATTTCCANGCCGCCCCCCG | 33 | Main chain of cnvK rC linker |
| EVQLLESGGGLVQPGGSLRLSCAAS | 34 | FR1 of DP-47 |
| WVRQAPGKGLEWVS | 35 | FR2 of DP-47 |
| RFTISRDNSKNTLYLQMNSLRAEDTAVYYCAK | 36 | FR3 of DP-47 |
| WGQGTLVTVSS | 37 | FR4 of DP-47 |

Here, for the sequence of FR3, the amino acid at the C-terminus of FR3 of the human framework sequence IGHV3-23*01 DP-47 can be changed to alanine (A), valine (V), arginine (R) and leucine (L) because the region can be involved in determination of the structure of the CDR3 region in VHH, and also involved in binding to an antigen. Amino acids at other positions can also be replaced with another amino acid. For example, the amino acid at the 13th, 31st or 32nd position from the N-terminus can be replaced with any amino acid selected from the group consisting of leucine (L), valine (V), alanine (A), asparagine (N), lysine (K) and arginine (R).

In this case, FR3 containing, at the above-described site, an amino acid to be replaced can be presented as an amino acid sequence represented by SEQ ID NO: 7. Here, X13 may be any amino acid selected from the group consisting of valine (V) and leucine (L), X31 may be any amino acid selected from the group consisting of alanine (A), asparagine (N) or lysine (K), and X32 may be an amino acid selected from the group consisting of alanine (A), valine (V), arginine (R) and leucine (L).

For example, the FR3 preferably has a sequence represented by an amino acid sequence selected from the group consisting of sequences of SEQ ID NOS: 8 to 31. This is because by performing the replacement in this way, the steric structure of the peptide can be controlled to some extent.

FR1 to FR4 may be chemically synthesized by a solid phase synthesis method or other routine methods, or their synthesis may be outsourced to companies.

### (2) Peptide constituting complementarity determining regions 1 to 3 (CDR1 to CDR3)

### (2-1) Length of CDR1 to CDR3

The peptide has three complementarity determining regions (hereinafter, abbreviated as "CDR") as described above, which may be, respectively, a complementarity determining region 1 (hereinafter, referred to as "CDR1") consisting of 8 to 12 amino acids, a complementarity determining region 2 (hereinafter, referred to as "CDR2") consisting of 14 to 18 amino acids, and a complementarity determining region 3 (CDR3) consisting of 6, 12 or 15 amino acids. It is preferable that CDR1 consist of 10 amino acids, CDR2 consists of 16 amino acids or 17 amino acids, and CDR3 consists of 6, 12 or 15 amino acids because a peptide having characteristics described later can be obtained.

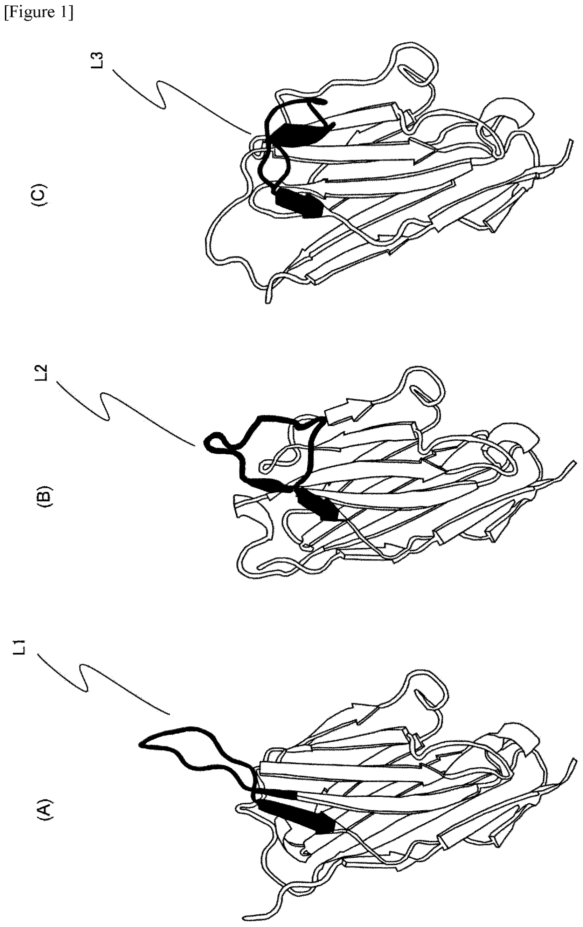

Since VHH has no light chain, FR2 is largely involved in formation of an antigen-binding site. CDR3 is classified roughly into three types according to distinctive loop structures L1 to L3 (Figures 1(A) to 1(C)). The loop structures L1 to L3 vary depending on the number of amino acids contained in CDR3, and influences the number of amino acid residues in CDR2. In the case of conventional antibodies consisting of a light chain and a heavy chain, FR2 is in contact with the light chain, whereas in VHH, the site is exposed. This is because VHH consists of only a light chain as described above.

### (2-2) Structures and classification of CDR1 to CDR3

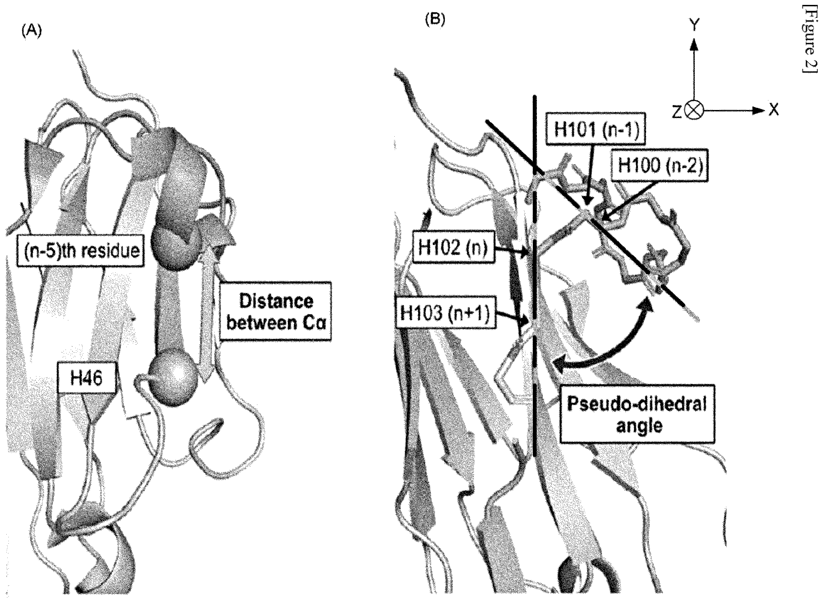

The structures of the three types of CDR3 are classified as follows according to three elements: a distance between the Cα atoms of an amino acid residue at a specific position which is contained in FR2 and an amino acid residue at a specific position which is contained in CDR3, an angle formed by two pseudo-planes made from the Cα atom of an amino acid residue at the position of the terminus of CDR3, and a structure of a loop itself (Figures 1(A) and 1(C) and Figures 2(A) and 2(B)). The criterial for the classification will be described below.

First, Cα refers to the first carbon atom adjacent to a functional group. Herein, the distance between the two α carbon atoms is referred to as a Cα distance. For example, when the amino acid at the C-terminus of CDR3 which is contained in the peptide corresponds to "n", the Cα distance is a distance between Cα(n-5) in the (n-5)th amino acid residue and Cα(46) in the 46th amino acid residue from the N-terminus of the peptide.

Second, the angle between the two pseudo-planes (hereinafter, referred to as a "dihedral angle") is defined as follows (Figure 2(B)). Figure 1(E) is a view of the peptide seen from an X-Y plane. Here, for example, when the 102nd amino acid residue from the N-terminus of the peptide corresponds to n (H102(n)), the first plane refers to a plane including a peptide bond contained in H102(n) and a peptide bond contained in an amino acid residue (H103(n+1)) adjacent to the amino acid residue (H102(n)) on the downstream side. Similarly, the second plane refers to a plane including a peptide bond contained in an amino acid residue (H101(n-1)) containing H101 and a peptide bond contained in an amino acid residue (H100(n-2)) adjacent to the amino acid residue (H101(n-1)) on the downstream side. The angle formed by the first plane and the second plane refers to a "dihedral angle".

Third, the shape of CDR3 itself can be classified into, for example, three types: an extended state in a Y direction as in L1 of Figure 1(A), a curled state as in L3 of Figure 1(C), and an intermediate state therebetween as in L2 of Figure 1(B). Note that CDR3 having a length of less than 6 amino acids is difficult to classify on the basis of the above-described criteria, but may be conveniently classified as the upright on the ground of its structural appearance.

### (2-3) Classification of peptide based on structural classification of CDR1 to CDR3

The upright shown in Figure 1(A) is defined as a type in which the Cα distance is longer than 15 Å, the dihedral angle is larger than 140 degrees, and the structure of CDR3 is in an extended state. The roll shown in Figure 1(C) is defined as a type in which the Cα distance is shorter than 10 Å, the dihedral angle is smaller than 140 degrees, and the structure of CDR3 is in a curled state. The half-roll shown in Figure 1(B) is defined as an intermediate structure between the upright and the roll.

By combining FR2, FR3, CDR2 and CDR3 of the upright type or the roll type shown in table 1, VHH having a desired structure, among the upright type, the roll type or the half-roll type, can be prepared.

Note that the structure of human VH can also be classified in the same manner as in the case of VHH.

A sub-library of the peptide can be constructed on the basis of the analyses of the antigen-binding site, the number of amino acid residues and the structure of CDR3. Here, the sub-library refers to a library prepared by selecting a clone from an original DNA library under specific conditions according to the purpose of study or the like.

### (2-4) Method for preparing peptide constituting CDR1 to CDR3

### (2-4-1) Source

A peptide constituting CDR1 to CDR3 can be prepared by selecting a target peptide. For example, structure data for VHH and human VH can be obtained as information on VHH from, for example, SAbDab Database (http://opig.stats.ox.ac.uk/webapps/newsabdab/sabdab/) in Protein Data Bank (PDB). Some selection criteria can be determined from the obtained various information on VHH, and VHH can be selected according to the criteria. Examples of the criteria for selection of VHH and human VH include those shown in Table 2 below.

**[Table 2]**

| No. | VHH selection criteria | Human VH selection criteria |
|---|---|---|
| 1 | Have Chain ID of heavy chain and no Chain ID of light chain | Have both Chain IDs of heavy chain and light chain |
| 2 | Tertiary structure determined by X-ray crystallographic analysis at a resolution of higher than 2.8 Å | Same as on the left |
| 3 | - | Heavy chain and light chain are those of Homo sapiens |
| 4 | Not complex with hapten antigen | Same as on the left |

CDR of VHH selected on the basis of the above criteria is determined by a Kabat method or another conventional method, for example. The CDR can be automatically determined using a commercially available tool, and examples of the tool include an ANARCI tool (http://opig.stats.ox.ac.uk/webapps/newsabdab/sabpred/anarci/).

The obtained information of amino acid residues can be used for defining a variable region and CDR, and clustering for grouping data on the basis of similarity between variable region (domain) data is performed. The clustering can be performed using CD-HIT (http://weizhong-lab.ucsd.edu/cd-hit/) or another clustering program. When CDR with a similarity (%id) of 100% indicating an exact match is removed during clustering, redundancy can be eliminated from the obtained cluster. Herein, the above process for eliminating redundancy is referred to as "curation".

From VHH and human VH obtained as a result of the curation described above, a sequence containing an amino acid sequence having a sufficient length as an antigen-binding site can be selected. If the length of the sequence obtained as described above has a length larger than that of the region determined by the Kabat method, for example, CDR1, a sequence obtained by combination with the sequence of a desired region prepared by a Chothia method, for example, CDR1 may be used as, for example, CDR1.

### (2-4-2) Designs of CDR1 and CDR2

For CDR1 and CDR2, instead of amino acid site-specific randomization, method for synthesizing and mixing sets of sequences that do not overlap with each other is used. This is intended to meet the evolution of germlines of antibody sequences in nature.

Amino acid sequences of CDR1 and CDR2 can be designed by the following procedure.

First, data obtained with a next-generation sequencer (which may be abbreviated as "NGS data" hereinafter) from a naive shuffling library for camelids, for example, alpacas, are used. It is preferable to use sequences concentrated to a certain level. The concentration ratio is not limited, but is, for example, preferably about 0.001% or more for the reason of reliability of data.

Subsequently, as an index of selecting candidate amino acid sequences of CDR1 and CDR2 from sequences obtained by concentrating the NGS data (hereinafter, referred to as "concentrated sequences"), amino acid sequences at BM positions in the concentrated sequences are used. This is intended to extract CDR1 and CDR2 of alpaca VHH with high structural stability.

Subsequently, it is preferable for the reason of tertiary structural stability that amino acid sequences with matching amino acid sequence at the BM position be selected from, for example, VHH sequences in PDB data, and taken as candidate amino acid sequences of CDR1 and CDR2.

The candidate amino acid sequences of CDR1 and CDR2 selected as described above are compared with the amino acid sequences of the germlines of alpacas. For example, with criteria established such that up to two mismatches for the sequence of CDR1 and up to three mismatches for the sequence of CDR2 are allowable, candidate sequences can be narrowed. In this way, sets for CDR1 and CDR2 can be provided.

### (2-4-3) Design of sequence of CDR3

For the sequence of CDR3, a library having a desired length can be designed on the basis of, for example, the distribution of the CDR3 loop length, a correlation between the CDR3 length and the structure classification, and the like. The CDR3 length may be made to vary between the upright and the roll, and appropriately selected so as to match the target peptide. For example, CDR3 may have a length of 6 amino acids or 12 amino acids for the upright, and CDR3 may have a length of 12 amino acids or 15 amino acids for the roll. As necessary, selection can be performed so that the ratios of 17 types of amino acids which do not include amino acids such as Cys, Met and Pro are equal. This is because the excluded amino acids may cause nonuniformity in the process of formulation of biological drugs.

### (3) Design of framework

The peptide of the present invention is a humanized heavy chain antibody derived from a camelid or the like, and therefore is required to have the framework part humanized.

Thus, first, a back mutation (BM) position in the peptide is determined on the basis of structure information available from a database such as that described above. A sequence, which is used as a standard in humanization of the framework region, is selected. Examples of the sequence include DP-47, DP-51 and DP-29 of the human framework sequence IGHV3-23*01. In studies on modification of the framework sequence, the amino acid sequences of Caplacizumab, Ozoralizmab, Vobaralizmab, other VHH and the like which are commercially available or used in clinical trial can be used as a reference.

Here, examples of the method for designing the humanized framework sequence include the following methods. A plurality of peptides from different sources are provided for comparison of their amino acid sequences. In the case where the results of comparison of the amino acid sequences show that amino acids assigned at the same position are the same, assigning those amino acids without change in the framework sequence according to the present invention is preferable for the reason that the antigenicity is not increased.

For example, the above-described four VHH amino acid sequences are selected for comparison, and if the results show that amino acids assigned at the same position are the same, those amino acids are assigned at the same position without change in the peptide of the present invention. Repeating this operation to determine amino acids to be assigned is preferable in design of a humanized framework.

Finally, if amino acids to be assigned cannot be determined, it is preferable that a correlation between the above-described structure classification of the CDR3 loop and a sequence profile, structure-related knowledge, knowledge of human framework sequences and the like be used in combination to perform the determination. For use as a medicament, it is preferable that in the process of preparing a humanized framework, some amino acids be replaced to reduce the occurrence frequency of, for example, cysteine and proline.

### (4) Construction of library

DNA fragments containing, respectively, CDR1 to CDR3 designed as described above are synthesized by overlap extension PCR, and a library comprising an intended full-length peptide can be constructed using a restriction enzyme and a ligase. Examples of the restriction enzyme used here include HphI, FokI, BsmBI, BtgZI and other Type IIS restriction enzymes (restriction enzymes that cleave a little distant site on one side by recognizing an asymmetric nucleotide sequence in which recognition and nuclease domains are separated), and examples of the ligase include T4 DNA ligase.

Primers for amplification of FR1 to FR4 (hereinafter, referred to as "FR3 primer", for example, in the case of FR3) can be designed so that a desired sequence is contained, and performing the design so that the FR4 primer contains a linker hybridization sequence for cDNA display is preferable for use of the cDNA display method. Examples of the desired sequence include a His tag region, 3' UTR, a T7 promoter, a SD sequence, and 5' UTR.

Primers for the sequences may be synthesized by a routine method, or their synthesis may be outsourced to an enterprise. Examples of the appropriate company include Eurofins Genomics K.K., and Sigma-Aldrich Co. LLC. For the reason of diversity of libraries, it is preferable that a large number of CDR1 primers different in the sequence of the CDR1 region and a large number of CDR2 primers different in the sequence of the CDR2 region be synthesized, mixed and used. It is preferable to use a trimer oligonucleotide for design of the CDR3 primer. This is because the occurrence frequency of amino acids can be controlled.

First, overlap extension PCR is performed under desired conditions to synthesize a DNA fragment containing each CDR. As a reaction liquid for overlap extension PCR, a solution, whose composition is shown in Table 3 below, can be used, the synthesized DNA fragments can be purified using, for example, AMPureXP (Beckman).

Next, the CDR1 fragment and the CDR2 fragment are treated with the restriction enzyme, purified, and then connected to each other with the ligase to obtain a ligation product (CDR1-2 fragment). The ligation product is purified by, for example, gel electrophoresis, and amplified using an outer primer. The amplified CDR1-2 fragment is purified, and the treated with a restriction enzyme again.

CDR3 is treated with a restriction enzyme and purified in the same manner as described above, and is then connected to the CDR1-2 fragment with a ligase. The obtained ligation product (CDR1-2-3 fragment) is purified in the same manner as described above, thereby obtaining a library. The sequence of the CDR1-2-3 fragment obtained as described above is confirmed using, for example, a next-generation sequencer.

It is possible to obtain a peptide library in the manner described above. By performing selection on the obtained peptide library by a cDNA display method using the following linker, an intended peptide can be obtained.

### (5) Preparation of cnvK Linker

From the viewpoint of reaction efficiency, the sequence of a biotin fragment that forms a main chain preferably has a sequence represented by SEQ ID NO: 33. Here, BioTEG is bound to the 5'-terminus of the main chain, and N at the 3-position in the nucleotide sequence denotes guanosine. To this sequence, CCT is further bound via Amino C6-dt. N at the 12-position denotes 3-cyanovinylcarbazole.

5'AANAATTTCCANGCCGCCCCCCG3' (SEQ ID NO: 33)

A puromycin segment that forms a side chain can have the sequence of 5'(5S)TcTCFCZZCC. P at the free terminus in the side chain sequence denotes puromycin as a protein-binding site. In addition, (5S) denotes 5' Thiol C6, c denotes cytidine, F denotes FITC-dT, and Z denotes Spacer 18. The chemical synthesis of the main chain and side chain may be outsourced to Eurofins Genomics K.K., Tsukuba Oligo Service Co., Ltd. or the like.

First, to 0.1 to 0.3 M sodium phosphate (pH 7.2), 10 to 20 nmol of a biotin fragment (final concentration 100 to 200 µM) and EMCS (manufactured by DOJINDO LABORATORIES, final concentration 13 to 19 mM) are added, and the mixture is incubated at 36°C to 38°C for 15 to 45 minutes. Thereafter, the obtained product is precipitated by adding ethanol. For the reaction, an appropriate commercially available kit, for example, Quick-Precip Plus Solution (manufactured by Edge BioSystems Inc.) may be used.

Next, a 35 to 40 nmol fraction of a puromycin segment is dissolved at a final concentration of 400 to 435 µM in a 0.5 to 1.5 M disodium hydrogen phosphate aqueous solution containing 25 to 75 mM of DTT, and the solution is stirred at room temperature for 0.5 to 1.5 hours using a shaker. Buffer exchange with a 0.01 to 0.03 M sodium phosphate buffer (pH 6.8 to 7.2) containing 0.02 to 0.04 M of NaCl is then performed. For this operation, a NAP5 column (manufactured by GE Healthcare Biosciences AB) or the like can be used.

The reduced puromycin segment solution after buffer exchange is mixed with the ethanol-precipitated product of the EMCS-modified biotin fragment, and the mixture is left to stand overnight at 2 to 6°C. Subsequently, DTT is added to the reaction liquid at a final concentration of 25 to 74 mM, the mixture is stirred at room temperature for 15 to 45 minutes, and ethanol precipitation is performed in the same manner as described above. The obtained ethanol-precipitated product is dissolved in 50 to 150 µL of Nuclease-free water (manufactured by Nacalai Tesque Inc.) to obtain a lysate.

The lysate is separated by, for example, 10 to 15% polyacrylamide gel electrophoresis, and cnvK rG Linker fractions are cut. The isolated gel may be crushed using BioMasher II Set (Nippi Inc.) or the like, followed by addition of 400 to 600 µL of Nuclease-free water, stirring of the mixture overnight at 2 to 6°C, and extraction of cnvK rG Linker. The stirred solution is transferred into an appropriate centrifuging tube filter, for example, a Costar (registered trademark) Spin-X (registered trademark) centrifuging tube filter (0.22 µm cellulose acetate (Corning Inc.)) or the like, and then centrifuged at 10,000 to 20,000 xg for 10 to 20 minutes to separate the gel and the extract. Thereafter, ethanol precipitation is performed as described above again. In this way, intended cnvK Linker can be obtained.

In formation of a mRNA/cDNA-linker-protein connected body, first, the above-described linker for preparation of a mRNA/cDNA-protein connected body and mRNA having a sequence complementary to the main chain of the linker are connected at a mRNA connecting site with T4 RNA ligase to form a mRNA-linker connected body. A protein is then synthesized from the mRNA in a cell-free translation system, and the synthesized protein is connected to the protein connecting site in the mRNA-linker connected body to form a mRNA-linker-protein connected body.

Subsequently, the mRNA-linker-protein connected body is bound to a solid phase via the solid phase binding site, and the solid phase to which the mRNA-linker-protein connected body is bound is washed with a first buffer. Thereafter, using the 3'-terminus of the main chain as a reaction initiation point and the mRNA as a template, a reverse transcription reaction is carried out to synthesize a cDNA chain, thereby obtaining a mRNA/cDNA-linker-protein connected body. The solid phase to which the mRNA/cDNA-linker-protein connected body is bound is washed with a second buffer, and the cleavage site of the main chain is cleaved with the predetermined endoribonuclease.

In the ligation reaction, the volume is 20 µL to 40 µL from the viewpoint of reaction efficiency, and the molar ratio of RNA to the linker is in the range of 3:1 to 1:6, and preferably 1:(1 to 2) (10 pmol to 20 pmol of the linker per 10 pmol of mRNA) for enhancing reaction efficiency and minimizing the remainder.

Next, using a heat block, an aluminum block, a water bath or another heating device, the sample solution is heated at about 60°C to 100°C for about 2 minutes to about 60 minutes, and then left to stand at room temperature for about 2 minutes to about 60 minutes, and the liquid temperature is slowly lowered. Thereafter, the sample solution is further cooled to about -5°C to about 10°C. Specifically, for example, the sample solution is heated on an aluminum block at 90°C for 5 minutes, then transferred onto an aluminum block at 70°C, and placed thereon for 5 minutes, and a photo-crosslinking reaction of the mRNA and the linker is then carried out. For example, the photo-crosslinking reaction is carried out by irradiation with 365 nm UV for 1 to 5 minutes in a 10 mM to 250 mM Tris-hydrochloride buffer containing 100 mM to 300 mM NaCl (pH 7.0 to 8.0).

As the cell-free translation system, an *Escherichia coli*-derived cell-free protein synthesis system is preferably used, and furthermore, an *Escherichia coli* reconstructing cell-free protein synthesis system is more preferably used.

For example, a translation reaction can be carried out using an *Escherichia coli* reconstructing cell-free protein synthesis system and the connected body in combination added thereto.

From the viewpoint of reaction efficiency, the amount of the *Escherichia coli* reconstructing cell-free protein synthesis system is set to about 8.5 µL to about 17 µL, the amount of the connected body is set to about 2.4 pmol to about 4 pmol, the size of the reaction system is set to about 12.5 µL to about 50 µL, and the reaction is carried out at about 20°C to about 40°C for about 10 minutes to about 30 minutes. Translation performed at about 37°C for about 30 minutes is associated with high formation efficiency and operation efficiency.

When after the translation reaction, a protein as a translated product and a mRNA-linker connected body are reacted at about 27°C to about 47°C for about 30 minutes to about 1.5 hours in the presence of, for example, about 0.3 M to about 1.6 M of potassium chloride and about 40 mM to about 170 mM of magnesium chloride (the concentration is a final concentration in all cases), the protein can be efficiently bound to the connected body.

Examples of the solid phase on which the mRNA-linker-protein connected body is fixed include beads such as styrene beads, glass beads, agarose beads, sepharose beads and magnetic beads; substrates such as glass substrates, silicon (quarts) substrates, plastic substrates and metal substrates (for example, metal foil substrates); containers such as glass containers and plastic containers; and membranes made from a material such as nitrocellulose or polyvinylidene fluoride (PVDF). In the case where the solid phase is formed of a plastic material such as styrene beads or a styrene substrate, a part of the linker may be covalently bound directly to the solid phase using a known method as necessary (manufactured by Qiagen N.V, see LiquiChip Applications Handbook or the like). In the case where biotin or an analog thereof is bound to the connected body, the connected body can be easily bound to the solid phase if avidin is bound to the solid phase in advance.

A connected body that has not been bound to a solid phase is removed by washing with, for example, a 1 mM to 100 mM Tris-hydrochloride buffer (pH 7.0 to 9.0) or phosphate buffer (pH 7.0 to 9.0) containing about 0.1 M to about 10 M of sodium chloride, about 0.1 mM to about 10 mM of EDTA and about 0.01% to about 1% of a surfactant. The use of a 20 mM Tris-hydrochloride buffer (pH 8.0) containing 2 M of sodium chloride, 2 mM of EDTA and 0.1% of Triton X-100 leads to good washing efficiency.

A cDNA chain is synthesized by carrying out a reverse transcription reaction under predetermined conditions using the 3'-terminus of the main chain as a reaction initiation point and the mRNA as a template. As a result, a mRNA/cDNA-linker-protein connected body is obtained. The reverse reaction system can be arbitrarily selected, but it is preferable to prepare a reaction system by adding the mRNA-linker-protein connected body, dNTP Mix, DTT, a reverse transcriptase, a standard solution, and water freed of RNase (hereinafter, referred to as "RNase-free water"), followed by reverse transcription in the system under conditions of 30°C to 50°C for 5 minutes to 20 minutes.

The free mRNA/cDNA-linker-protein connected body is then removed by performing washing with the same buffer as described above. Thereafter, the cleavage site of the main chain is cleaved with the endonuclease as described above. In the manner described above, various proteins, as well as cDNAs corresponding to the proteins can be obtained using the linker according to the present invention.

In the above method, the obtained mRNA and linker DNA having puromycin are fixed on streptavidin-modified magnetic beads using an avidin-biotin bond, a protein is synthesized from mRNA using a cell-free translation system, and cDNA is synthesized from mRNA using a reverse transcription reaction. Thus, proteins as a phenotype and DNA sequence information as a genotype are mapped on a one-to-one basis on magnetic beads.

Subsequently to the step of cleavage and separation, a mRNA/cDNA-linker-protein connected body can be selected by utilizing, for example, the affinity of the obtained protein. Thereafter, a variation is introduced into a nucleotide sequence in the selected connected body and an amplification reaction is carried out by a PCR method or the like. By a predetermined method, the amplification product is connected to double-stranded DNA having a desired promoter sequence, thereby obtaining first-generation variant mRNA (hereinafter, abbreviated as "mRNA G1"). By subsequently repeating the steps of the cDNA display method, as described above, using mRNA G1, mRNA G2, mRNA G3 and the like can be obtained, and by introducing a desired mutation or the like while repeating the above reaction, peptide aptamers having various sequences can be obtained.

### Examples

### (Example 1) Analysis of structure of VHH CDR3

First, structure data for VHH and human VH were obtained from SAbDab Database (http://opig.stats.ox.ac.uk/webapps/newsabdab/sabdab/) in Protein Data Bank (PDB). VHH obtained here was selected on the basis of the criteria shown in Table 2 above except for VHH in which CDR has a short 5-amino acid terminus structure.

CDR sequence of VHH obtained from the database was determined by a Kabat method which is a conventional method. In the present Example, using an ANARCI tool (http://opig.stats.ox.ac.uk/webapps/newsabdab/sabpred/anarci/), CDR determination was automatically performed by the Kabat method to obtain the information of numbered amino acid sequences. The obtained information was used to define variable regions and CDR.

On the basis of amino acid sequences contained in the information, clustering was performed on variable domain regions using CD-HIT (http://weizhong-lab.ucsd.edu/cd-hit/), followed by curation in which exactly matching parts with %id being 100% were deleted to eliminate redundancy.

As a result of the curation, 330 sequences of VHH and 926 sequences of human VH were obtained.

The results of the analysis revealed that CDR2 (H50-H65) and CD3 (H95-H102) contains sufficient antigen-binding sites. However, the actual antigen-binding site in CDR1 was broader than the region (H31-H35) determined by the Kabat method, which was therefore adjusted to CDR1 (H26-H35) by combination with CDR1 (H26-H32) determined by a Chothia method.

As described above, as a result of classifying CDR3 roughly into three types according to its distinctive loop structures (Figure 1), it has become evident that the loop structures depend on the number of amino acids contained in CDR3 and the number of amino acid residues in CDR2 as described above.

It has become evident that in conventional antibodies consisting of a light chain and a heavy chain, FR2 is in contact with the light chain, whereas in VHH, FR2 is exposed at a site which is in contact with the light chain, and accordingly, FR2 of VHH contributes significantly to formation of an antigen-binding site. In Figure 1, amino acid residues which are near the antigen are indicated in black.

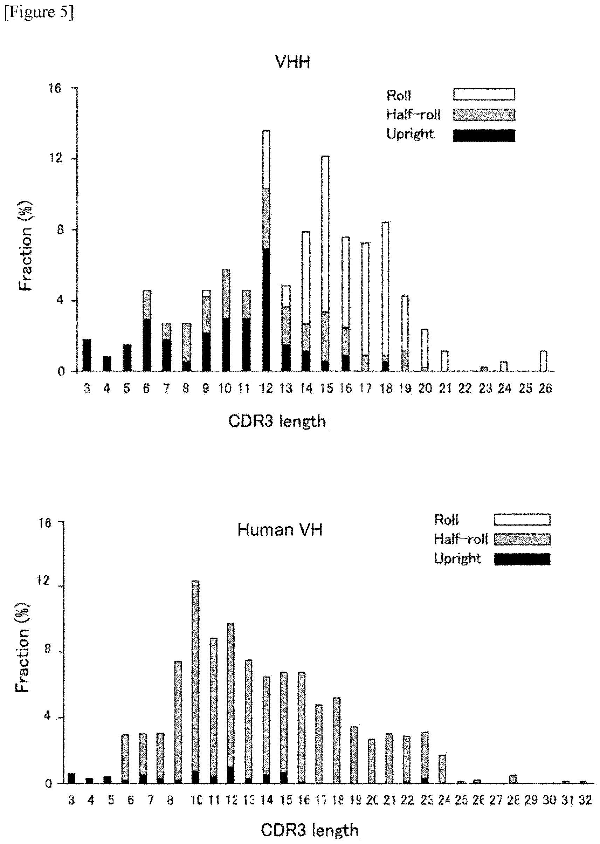

The three types of loop structures of CDR3 were defined as follows. A loop structure in which the Cα distance is 15 Å or more and the dihedral angle is 140 degrees or more was defined as an upright type, a loop structure in which the Cα distance is 10 Å or more and the dihedral angle is 140 degrees or less was defined as a roll type, and a loop structure in which the Cα distance is 15 Å or more and the dihedral angle is less than 140 degrees or the Cα distance is more than 10 Å and the dihedral angle is less than 140 degrees is defined as a half-roll type. Figure 5 shows a relationship between the length of CDR3 and the relevant classification type. From this, it has been seen that CDR3 having a length of less than 12 amino acids tends to be classified as the upright type, and CDR3 having a large length of 12 amino acids or more tends to be classified as the roll type. It has also been found that the half-roll type has no relation with the CDR3 length.

It has been found that CDR3 classified as the upright type tends to contain a framework region at an antigen-binding site. On the other hand, it has also been shown that CDR3 containing 14 or more amino acids is more likely to be classified as the roll type, and less likely to contain a framework region at an antigen-binding site. Further, it has become evident that the roll structure is stabilized as the framework region and the CDR3 region become closer to each other. Further, it has been found that in the case where 16 or more amino acids are contained in CDR3, the population of CDR3 containing cysteine increases, and the cysteine forms a disulfide bond with cysteine of CDR2 to form more stable CDR3.

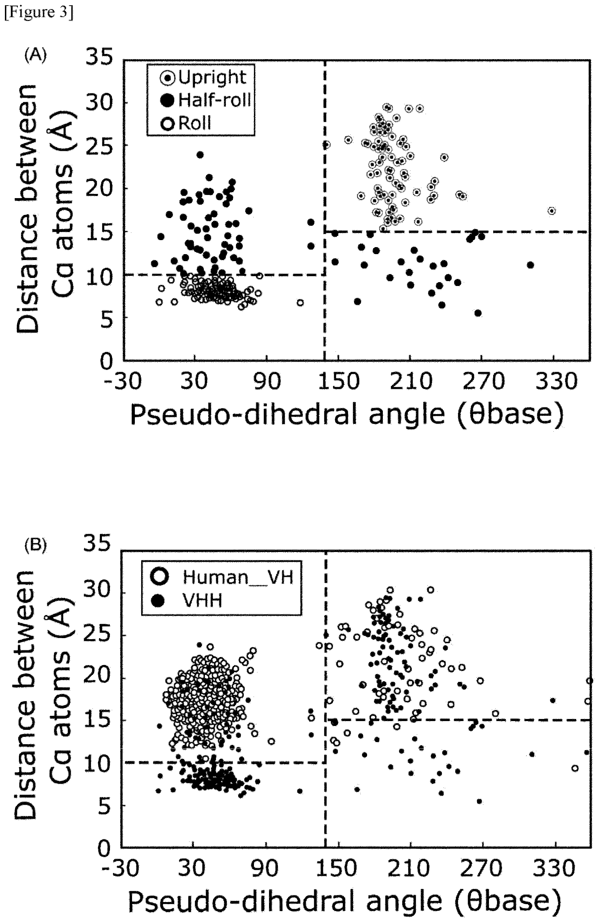

For human VH, the structure was classified in the same manner as in the case of VHH. Figure 3(B) shows a two-dimensional plot of the Cα distance versus the dihedral angle. In the case of human VH, most were classified as the half-roll type, none was classified as the roll type, and few were classified as the upright type. From this, it has been thought that in the human antibody, the presence of a light chain poses a steric obstruction to prevent CDR3 from being folded into the structure such as a roll type.

### (Example 2) Construction of artificial VHH library

### (2-1) Library design based on analysis data

A reasonable artificial VHH library comprising four sub-libraries: Upright 6, Upright 12, Roll 12 and Roll 15 was designed on the basis of the analysis results for the above-described antigen-binding site, the number of amino acid residues and the structure of CDR3. CDR2 of upright type sub-library was formed from 16 amino acid residues, and CDR2 of roll type sub-library was formed from 17 amino acid residues. This is because a strong correlation between the number of amino acid residues constituting CDR2 (16 amino acid residues or 17 amino acid residues) and the formation of the upright type or roll type was confirmed.

The amino acid sequence of CDR was designed so that CDR1 consisted of 150 types of sequence sets, CDR2 consisted of 69 or 71 types of sequence sets, and CDR3 consisted randomly of 17 types of amino acids which do not include three types: methionine, proline and cysteine. CDR3 having a length of 16 amino acids or more was not designed. This is because additional formation of a disulfide bond is not desirable from the pharmaceutical point of view. Finally, the CDR1 to CDR3 designed as described above were, as one set, incorporated into humanized FR.

In view of the above analysis results, the framework regions and CDR of VHH in the present library were defined as follows: FR1 (H1-H25), CDR1 (H26-H35), FR2 (H36-H49), CDR2 (H50-H65), FR3 (H66-H94), CDR3 (H95-H102) and FR4 (H103-H113). Next, framework sequences were designed on the basis of the above definitions.

### (2-2) Design of framework

First, back mutation (BM) positions in VHH were determined on the basis of the structure information. Using DP-47 of the human framework sequence IGHV3-23*01 as a standard, the framework region was humanized with reference to the amino acid sequences of three VHHs that are commercially available or used in clinical trial (Caplacizumab, Ozoralizmab and Vobaralizmab).

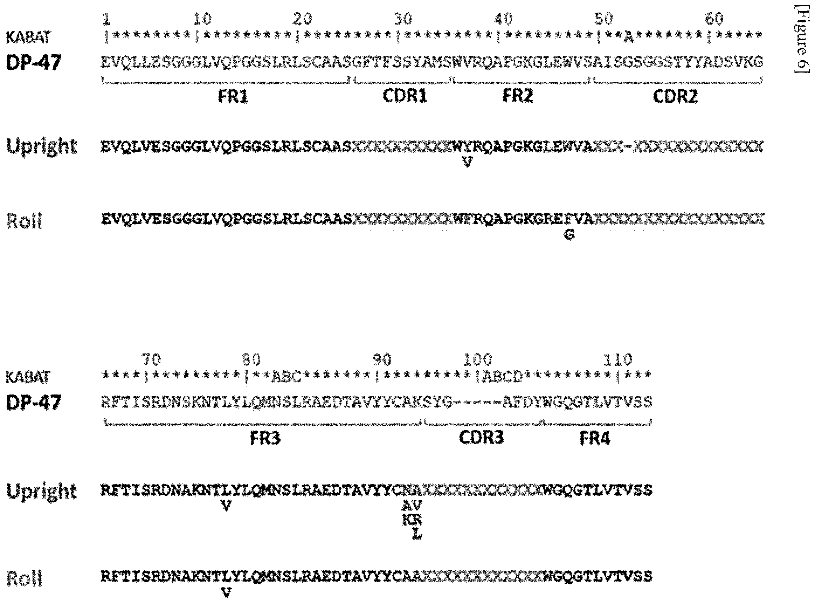

Here, in the case where the four VHH amino acid sequences indicate the same amino acid, this amino acid was determined. For amino acid sequences that had not been determined, a correlation between the structure classification of the CDR3 loop and a sequence profile, structure-related knowledge, knowledge of human framework sequences and the like were used in combination to determine the amino acid sequences (Figure 6). Some amino acids were further edited in the humanization process to reduce the occurrence frequency of amino acids that are not desirable from the pharmaceutical point of view.

### (2-3) Design of CDR1 and CDR2

Since CDR1 and CDR2 are unlikely to deviate substantially from the germlines of antibody sequences in the natural world, not amino acid site-specific randomization, but a method for synthesizing and mixing CDR sequences distinct from one another was used. In practice, the amino acid sequences of CDR1 and CDR2 were designed by the following procedure.
(p1) Sequences concentrated to above a certain level (ratio = 0.001% or more, hereinafter referred to as "concentrated sequences"), out of the NGS data of the alpaca-derived Naive Shuffling Library, was used as data.
(p2) From the concentrated sequences, those whose amino acid sequence at the BM position is identical to the amino acid sequence at the BM position in the FR sequence designed in (2-2) above were selected, and listed as candidate amino acid sequences of CDR1 and CDR2.
(p3) VHH sequences whose amino acid sequence at the BM position is identical to the amino acid sequence at the BM position in the FR sequence were selected out of data registered in the Protein data bank (PDB) (which may be referred to as "PDB data" hereinafter), and were listed as candidate amino acid sequences of CDR1 and CDR2.
(p4) The CDR1 and CDR2 sequences listed in (p2) and (p3) above were compared to the germline sequences of the alpaca, and the candidate sequences were narrowed to allow up to two mismatches in the case of CDR1 and up to three mismatches in the case of CDR2, and added to the sequence sets in (p2) above.

### (2-4) Design of CDR3

Here, in correlation to the distribution of the CDR3 loop length, the CDR3 length and the structure classification of CDR3, a total of 4 types of sub-libraries with a CDR3 length of 6 amino acids or 12 amino acids for the upright type and a CDR3 length of 12 amino acids or 15 amino acids for the roll type were designed. The sub-libraries were designed so that the ratios of 17 types of amino acids which do not include three amino acids (Cys, Met and Pro) that may cause nonuniformity in the process of formulation were equal.

### (2-5) Construction of library by DNA assembly

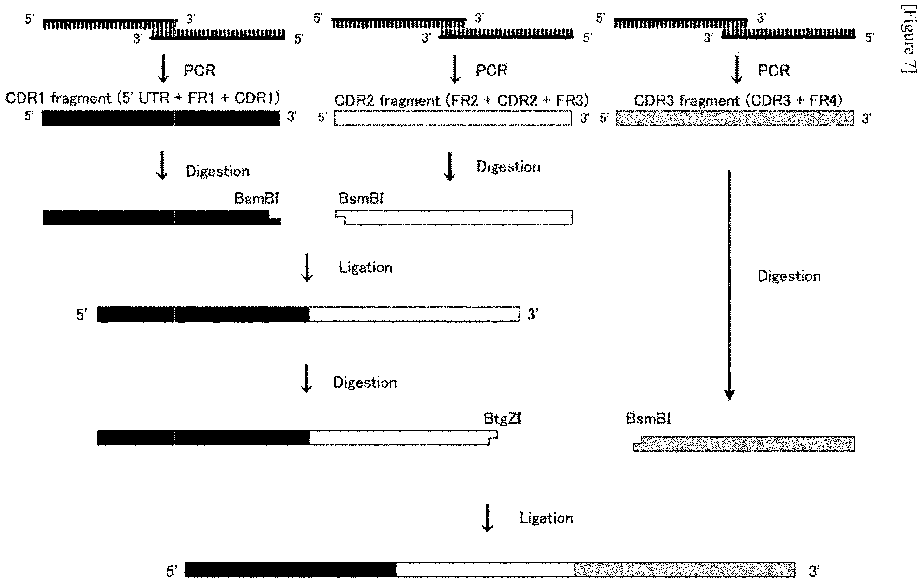

DNA fragments comprising one each of CDR1 to CDR3, respectively, were synthesized by overlap extension PCR, and the fragments were ligated using Type IIS restriction enzyme and T4 DNA Ligase, thereby constructing a library comprising an intended full-length VHH sequence (Figure 7).

The synthesis of primers corresponding to FR3 and FR4 was outsourced to Eurofins Genomics K.K. As the FR4 primer, one containing a His tag region and a 3' UTR (containing a linker hybridization sequence in cDNA display) was designed. The synthesis of primers corresponding to FR1, CDR1 and CDR2 was outsources to Sigma-Aldrich Co. LLC. As the FR1 primer, one containing 5' UTR (containing T7 promoter and a SD sequence) was designed. As the CDR1 primer, 150 types of CDR1 primers different in the sequence of the CDR1 region were each synthesized, and as the CDR2 primer, 140 types of CDR2 primers different in the sequence of the CDR2 region (Upright type: 71 types, Roll type: 69 types) were each synthesized. These primers were mixed and used. A primer corresponding to CDR3 in each library was designed so as to control the occurrence frequency of the amino acid using a trimer oligonucleotide, and its synthesis was outsourced to Ella Biotech GmbH.

First, DNA fragments containing each CDR were synthesized, where by overlap extension PCR, a CDR1 fragment was synthesized using the FR1 primer and the CDR1 primer, a CDR2 fragment was synthesized using the FR2 primer and the CDR2 primer, and a CDR3 fragment was synthesized using the CDR3 primer and the FR4 primer. The reaction composition was set to 125 µL of PrimeSTAR MAX PreMIX (2×) (Takara Bio Inc.), 5 µL of each of 10 µM forward/reverse primers and 115 µL of ultrapure water. However, these amounts were all doubled in the synthesis of the CDR3 fragment. Table 3 shows the reaction conditions. The synthesized DNA fragments were purified using AMPureXP (manufactured by Beckman Company) (protocols in written instructions were followed; this method is used whenever merely "purification" is mentioned hereinafter).

**[Table 3]**

| Item | Initial denaturation | Denaturation | Annealing | Extension | Final extension | Cooling |
|---|---|---|---|---|---|---|
| Temperature (°C) | 98 | 98 | 65 | 72 | 7 | 16 |
| Time (sec) | 60 | 10 | 5 | 10 | 60 | ∞ |
| Cycle number | 1 | 5 | | | 1 | 1 |

Next, the CDR1 fragment and the CDR2 fragment were treated with a restriction enzyme at 55°C for 15 minutes using BsmB I (NEB). The DNA fragments treated with a restriction enzyme were purified by a routine method, and ligated at 16°C for 16 hours with T4 DNA Ligase (Takara Bio Inc.) to synthesize a connected body of the fragments, i.e., a CDR1-CDR2 fragment, as a ligation product. The ligation product was migrated at 200 V for 40 minutes with 8 M urea PAGE-gel to purify an intended product region, and PCR amplification was then performed 10 cycles at an annealing temperature of 62°C using an outer primer. The amplified CDR1-2 fragment was purified, and then treated with a restriction enzyme at 60°C for 15 minutes using BtgZ I (NEB).

The CDR3 fragment was treated with a restriction enzyme at 55°C for 15 minutes using BsmBI, and purified with AMPure XP to obtain a purified CDR3 fragment. Next, the purified CDR1-2 fragment and the purified CDR3 fragment were ligated at 16°C for 16 hours with T4 DNA Ligase to obtain a full-length VHH sequence. The ligation product was purified with the 8 M urea-denatured PAGE gel, and collected to construct a library.

### (2-5) Confirmation of nucleotide sequence and amino acid sequence in prepared library

For confirming whether the library constructed in (2-4) above had a nucleotide sequence and an occurrence frequency of an amino acid consistent with the design, the nucleotide sequence and the amino acid sequence in the library were analyzed using a next-generation sequencer (NGS Inc.).

First, PCR amplification was performed on the above-described libraries. The composition of the reaction solution (total 25 µL) was set to 12.5 µL of PrimeSTAR MAX PreMIX (2×), 1 µL of a library sample, 0.5 µL of each of 20 µM forward/reverse primers (PL_prRd-N4_NL_FW, PL_prRd-N4_NL_RV), and 10.5 µL of ultrapure water. The reaction conditions were the same as in Table 3 above except that the annealing temperature was 62°C, the extension time was 5 seconds, and the cycle number for denaturation was 8.

The obtained PCR product was purified using AMPureXP, and Index PCR was performed using the purified product as a template (reaction conditions are the same as the PCR conditions except that the annealing temperature was changed to 52°C). The composition of the reaction solution (total 25 µL) was set to 12.5 µL of PrimeSTAR MAX PreMIX (2×), 1 µL of the PCR product, 2 µL of each of 5 µM forward/reverse primers (Nextera XT Index 1 Primers (N7XX), Nextera XT Index 1 Primers (S5XX)), and 7.5 µL of ultrapure water. The Index PCR product was purified using AMPure as described above, and the DNA concentration was measured using NanoPad DS-11. Thereafter, the Index PCR purified product was diluted to a concentration of 10 nM, and the products were then collected together in one tube in an amount of 5 µL per product, and mixed.

In NGS here, analysis was performed using MiSeq Reagent Kit v3 (600-cycle) (Illumina, Inc.). A reagent cartridge was dissolved in deionized water. A 10 nM NGS sample library was diluted to a 4 nM library with a resuspension buffer. 5 µL of 0.2 M NaOH was added to 5 µL of the 4 nM library, the mixture was vortexed, and reacted at room temperature for 5 minutes, and 990 µL of ice-cooled HT1 was then added to prepare a 20 pM library. To 450 µL of the 20 pM library, 150 µL of ice-cooled HT1 was added to prepare a 15 pM sample library. A PhiX library to be used as a control DNA library was similarly prepared as a 15 pM library. Finally, 570 µL of the 15 pM sample library and 30 µL of the 15 pM PhiX library were mixed, the reagent cartridge was loaded with the ultimate NGS sample library, and the reagent cartridge was set in Miseq, followed by analysis. The obtained data was analyzed to confirm that each library had a nucleotide sequence consistent with the design.

### (Example 3) Acquisition of antigen-binding VHH clone from PharmaLogical Library

### (3-1) Preparation of linker for in vitro selection

A cnvK linker for selection was prepared as follows.

### (1) Preparation of cnvK rC Linker

The sequence of a biotin fragment that forms a main chain was a sequence represented by SEQ ID NO: 33 below. Here, BioTEG is bound to the 5'-terminus of the biotin fragment (main chain), and in the nucleotide sequence, N at the 3-position denotes guanosine, and N at the 12-position denotes 3-cyanovinylcarbazole. To the 3'-terminal of the sequence of SEQ ID NO: 33 below, CCT was bound via Amino C6-dT.

5'AANAATTTCCANGCCGCCCCCCG3' (SEQ ID NO: 33)

A puromycin segment that forms a side chain has the sequence of 5'(5S)TcTCFCZZCCP. P at the free terminus in the side chain sequence denotes puromycin as a protein-binding site. In addition, (5S) denotes 5' Thiol C6, c denotes cytidine, F denotes FITC-dT, and Z denotes Spacer 18. The chemical synthesis of the main chain and the side chain was outsourced to Tsukuba Oligo Service Co., Ltd.

First, to 0.2 M sodium phosphate (pH 7.2), 15 nmol of the biotin fragment (final concentration 150 µM) and EMCS (manufactured by DOJINDO LABORATORIES, final concentration 16.7 mM) were added, and the mixture was incubated at 37°C for 30 minutes, and then ethanol precipitated using Quick-Precip Plus Solution (Edge BioSystems Inc.).

Next, a 37.5 nmol fraction of the puromycin segment was dissolved at a final concentration of 417 µM in a 1 M disodium hydrogen phosphate aqueous solution containing 50 mM of DTT, and the solution was stirred at room temperature for 1 hour using a shaker. Buffer exchange to a 0.02 M sodium phosphate buffer (pH 7.0) containing 0.03 M of NaCl was then performed using a NAPS column (manufactured by GE Healthcare Biosciences AB).

The reduced puromycin segment solution after buffer exchange was mixed with an ethanol-precipitated product of the EMCS-modified biotin fragment, and the mixture was left to stand overnight at 4°C. Subsequently, DTT was added to the reaction liquid at a final concentration of 50 mM, and the mixture was stirred at room temperature for 30 minutes. Thereafter, ethanol precipitation was performed using Quick-Precip Plus Solution (manufactured by Edge BioSystems Inc.). The ethanol-precipitated product was dissolved in 100 µL of Nuclease-free water (manufactured by nacalai tesque).

The lysate was separated by 12% polyacrylamide gel electrophoresis, and cnvK rC Linker fractions were isolated. The isolated gel was crushed using BioMasher II Set (Nippi Inc.), 500 µL of Nuclease-free water was added, the mixture was stirred overnight at 4°C, and cnvK rC Linker was extracted. The stirred solution was transferred into a Costar (registered trademark) Spin-X (registered trademark) centrifuging tube filter, 0.22 µm cellulose acetate (Corning Inc.), and then centrifuged at 16,000 xg for 15 minutes to separate the gel and the extracted liquid. Thereafter, ethanol precipitation was performed using Quick-Precip Plus Solution, thereby obtaining intended cnvK rC Linker. The obtained cnvK rC Linker was dissolved in Nuclease-free water, and stored at -20°C.

### (3-2) In vitro selection

In vitro selection was performed to examine whether or not VHH binding to various antigens was obtained from the constructed PharmaLogical library.

Antigens used here as a target were HSA (human serum albumin), HER2 (human epidermal growth factor receptor 2), GPC3 (Glypican 3) and GPC1 (Glypican 1). As a library for selection, one obtained by mixing the above-described four types of sub-libraries was used. The series of operations of synthesis of a mRNA-linker connected body, synthesis of a cDNA display molecule, in vitro selection and PCR amplification were defined as one round, and three rounds of selection (four rounds for GPC1) were performed (Figure 7).

### (3-2) Synthesis of mRNA-linker connected body

In the first round, using 10 pmol of the DNA library in each sub-library as a template, a transcription reaction was carried out at 37°C for 30 minutes using T7 RiboMAX Express Large Scale RNA Production System (manufactured by Promega Corporation). After the transcription reaction, RQ1 RNase-free DNase (manufactured by Promega Corporation) was added, the mixture was reacted at 37°C for 15 minutes to degrade DNA, and RNA was purified using RNAclean XP (manufactured by Beckman Company) (protocols in written instructions were followed). Thereafter, the RNA concentration was measured using NanoPad DS-11, reagents were mixed at a final concentration of 200 mM for NaCl, 50 mM for Tris-HCl (pH 7.5), 1 µM for mRNA, and 1 µM for cnvK linker, and the mixture was heat-treated with the following program: (s1) at 90°C for 1 minute, then (s2) at 70°C for 1 minute and then (s3) at 4°C, to hybridize a linker for synthesis of cDNA display to mRNA. Next, using UVP closslinker cl-3000, the mRNA-linker hybridized product was irradiated with UV at 365 nm to a total energy amount of 406 mJ/cm² to photo-crosslink the mRNA and the linker, thereby preparing a mRNA-linker connected body.

### (3-3) Synthesis of cDNA display molecule

The mRNA-linker connected bodies of the sub-libraries were mixed to obtain a mixture. Using the mixture as a template, a cell-free translation reaction (which may be referred to as "IVV" hereinafter) was carried out at 37°C for 30 minutes using a reconstructing cell-free translation solution Purefrex 1.0 (Gene Frontier Corporation) and a DsbC set (Gene Frontier Corporation) as a SS binding auxiliary factor. The composition of the reaction liquid was set to 1,600 µL of solution I, 160 µL of each of solution II and solution III, 800 µL of a 1 pmol/µL ligation product, 160 µL of a DsbC set diluted 4-fold, 160 µL of 20 mM GSSG and 160 µL of 80 mM GSH. After the translation, 1,920 µL of an IVV formation buffer was added, and the mixture was then reacted at 37°C for 30 minutes to covalently bind the translated product to the linker via puromycin. Subsequently, 1,280 µL of 0.5 M EDTA was added, and the mixture was reacted at 4°C for 10 minutes to separate ribosome from the nucleic acid.

By the above operations, 6,400 µL of an IVV product (mRNA-linker-VHH conjugate) was obtained. The obtained IVV product was purified in accordance with the attached protocols using 3,840 µL of Dynabeads Streptavidin MyOne C1 (manufactured by Thermo Fisher Scientific). To the beads to which the IVV product was bound, a reverse transcription reaction solution comprising 1,280 µL of a 5 × RT buffer, 256 µL of 25 mM dNTPs, 128 µL of reverse transcriptase and 4,736 µL of ultrapure water was then added, and the mixture was reacted at 42°C for 1 hour to synthesize a cDNA display product (cDNA-mRNA-linker-peptide conjugate).

The cDNA display product obtained as described above was eluted from the beads with 640 µL of an elution buffer containing RNase T1 (manufactured by Thermo Fisher Scientific). Thereafter, using 20 µL of His-Mag Sepharose (manufactured by Cytiva Company), His tag purification was performed on the cDNA display product to obtain 200 µL of a cDNA display molecule (cDNA-linker-peptide connected body).

### (3-4) Selection by exclusion in test tube

The cDNA display molecule and a biotinylated target protein (HSA, HER2, GPC3, GPC1) were mixed in amounts shown in Table 4, and the mixture was incubated at 4°C for 30 minutes. Thereafter, the reaction liquid was mixed with 120 µL of washed Dynabeads Streptavidin MyOne C1, and the mixture was incubated at 4°C for 30 minute, and the binding cDNA display molecules were immobilized on beads via the biotinylated target protein.

The coated beads (cDNA display-biotinylated target protein streptavidin-beads) were washed with 200 µL of PBS-T three times (the tube was replaced before the third washing), then eluted twice with 100 µL of an elution buffer, and only nucleic acid regions of the cDNA display molecule, which have ability to bind to the target protein, were collected. The obtained eluted sample was purified using AMPure XP.

In the present Example, in vitro selection was performed three or four rounds. In the second and third rounds, the mixing was performed with the composition set to 20 µL of a cDNA display solution, 10 µL of a 1 mM biotinylated target protein, 2.5 µL of a 200 mg/mL heparin solution, 0.5 µL of 10% Tween and 67 µL of 1 × PBS so that the concentration of each target protein was 100 nM. In the case where the target protein other than HSA was used, BSA (FUJIFILM Wako Pure Chemical Corporation) was added at a final concentration of 0.5% for excluding non-specific binding molecules. In the case where HAS was used, selection was carried out without adding BSA. The beads were coated with MyOne T1 in the second round and with MyOne C1 in the third round. As described above, changing the surface characteristics of the beads used for each round was aimed at exclusion of cDNA display molecules binding to the beads in a non-specific manner (see Table 4 below).

**[Table 4]**

| Round | mRNA/linker connected product (Ligation ; pmol) | Target protein concentration (nM) | Beads used and its amount (µL) |
|---|---|---|---|
| 1^{st} | 400 | 500 | MyOne C1 : 120 |
| 2^{nd} | 12.5 | 100 | MyOne T1 : 21 |
| 3^{rd} | 12.5 | 100 | MyOne C1 : 24 |

### (3-4) PCR amplification of selected sample

Using PrimeSTAR MAX (Takara Bio Inc.), PCR amplification was performed on DNA eluted in the selection. In the first round, quantitative PCR was not performed, and PCR was performed using the total amount of eluted sample as a template. The composition of the reaction solution (total 250 µL) was set to 125 µL of PrimeSTAR MAX PreMIX (2×), 100 µL of the eluted DNA sample, 5 µL of each of 20 µM forward/reverse primers (PL_T7pro, NewYtag_cnvk-linker), and 15 µL of ultrapure water. The PCR program was the same as in Table 3 except that the annealing temperature was 60°C, the extension time was 5 seconds, and the cycle number for denaturation or the like was 15. The PCR product was purified using AMPure XP.

In the second and third rounds, a part of the eluted sample was used for quantitative PCR, and the rest was used as a template for PCR amplification. The composition of the solution used for quantitative PCR (total 25 µL) was set to 12.5 µL of THUNDERBIRD qPCRMix (TOYOBO CO., LTD.), 0.5 µL of each of 20 µM forward/reverse primers (PL_T7pro, NewYtag_cnvk-linker), 1 µL of eluted sample DNA and 10.5 µL of ultrapure water. The PCR program was such that after the initial denaturation at 98°C for 30 seconds, the cycle of denaturation at 98°C for 5 seconds, annealing at 60°C for 10 seconds and extension at 72°C for 35 seconds was repeated 35 times.

Subsequently, PCR amplification was performed by reference to the Ct value obtained as data. The composition of the reaction solution (total 100 µL) was set to 50 µL of PrimeSTAR MAX PreMIX (2×), 25 µL of the eluted DNA sample, 2 µL of each of 20 µM forward/reverse primers (PL_T7pro, NewYtag_cnvk-linker), and 21 µL of ultrapure water. The program was the same as the program shown in Table 3 except that the annealing temperature was 65°C, the extension time was 5 seconds, and the cycle number for denaturation or the like was 15.

### (Example 4) Analysis of selection product by NGS

### (4-1) Preparation of sequence sample

First, using the concentrated libraries of target molecules obtained by the selection, PCR amplification was performed under the following conditions. The composition of the reaction liquid (total 25 µL) was set to 12.5 µL of PrimeSTAR MAX PreMIX (2×), 1 µL of the selection product, 0.5 µL of each of 20 µM forward/reverse primers (PL_prRd-N4_NL_FW, PL_prRd-N4_Ytag_RV), and 10.5 µL of ultrapure water. The PCR program was the same as in Table 3 except that the annealing temperature was 62°C, the extension time was 5 seconds, and the cycle number for denaturation or the like was 8.

The obtained PCR product was purified using AMPure XP, and Index PCR was performed using the purified product as a template. The composition of the reaction liquid (total 25 µL) was 12.5 µL of PrimeSTAR MAX PreMIX (2×), 1 µL of the selection product, 0.5 µL of each of 5 µM forward/reverse primers (Nextera XT Index 1 Primers (N7XX), Nextera XT Index 2 Primers (S5XX)), and 10.5 µL of ultrapure water. The PCR program was the same as in Table 3 except that the annealing temperature was 52°C, the extension time was 5 seconds, and the cycle number for denaturation or the like was 8. The Index PCR product was similarly purified using AMPure XP, and the DNA concentration was measured using NanoPad DS-11. The Index PCR purification product was diluted to 10 nM, and the products were then collected together in one tube in an amount of 5 µL per product, and mixed.

### (4-2) Procedure of NGS

In NGS here, analysis was performed using MiSeq Reagent Kit v3 (600-cycle) (Illumina, Inc.). A reagent cartridge was dissolved in deionized water. A 10 nM NGS sample library was diluted to a 4 nM library with a resuspension buffer. 5 µL of 0.2 M NaOH was added to 5 µL of the 4 nM library, the mixture was vortexed, and reacted at room temperature for 5 minutes, and 990 µL of ice-cooled HT1 was then added to prepare a 20 pM library. To 450 µL of the 20 pM library, 150 µL of ice-cooled HT1 was added to prepare a 15 pM sample library. A PhiX library to be used as a control DNA library was similarly prepared as a 15 pM library. Finally, 570 µL of the 15 pM sample library and 30 µL of the 15 pM PhiX library were mixed to obtain an ultimate NGS sample library. The reagent cartridge was loaded with the ultimate NGS sample library, and the reagent cartridge was set in Miseq, followed by analysis. The obtained NGS analysis data was processed to identify a binding sequence for each target molecule.

### (Example 5) Evaluation of primary binding in VHH

### (5-1) Preparation of VHH expression plasmid library

The DNA library encoding VHH selected by the in vitro selection was cloned into a plasmid vector for VHH expression. First, to the DNA library obtained by the in vitro selection, a sequence to be treated with a restriction enzyme is added by PCR to obtain a PCR product. The PCR product and a C. glutamicum expression plasmid vector were treated with restriction enzyme BamHI at 37°C for 1 hour, and then with restriction enzyme SfiI at 50°C for 1 hour. The VHH DNA product corresponding to Insert was purified with AMPure XP, and the obtained Vector DNA product was subjected to agarose gel electrophoresis at 100 V for 30 minutes, thereby being isolated and purified.

The Vector DNA purified as described above was subjected to a dephosphorylation reaction at 37°C for 1 hour using FastAP Thermosensitive Alkaline Phosphatase (manufactured by Thermo Fisher Scientific) which is a dephosphorylation enzyme. Thereafter, the Insert DNA and the Vector DNA were mixed at a molar ratio of 1: 10, and a ligation reaction was carried out overnight at 16°C using Ligation high (TOYOBO CO., LTD.), thereby obtaining a plasmid library containing a selected VHH library.

### (5-2) Production of VHH (culture supernatant)

Each plasmid library was introduced into C. glutamicum by electroporation to obtain a transformant. The obtained transformant was inoculated into CM2G culture medium, and cultured overnight at 30°C. Thereafter, the culture solution was passed to PM1S culture medium as a culture medium for VHH expression, and cultured at 25°C for 72 hours to secret and express VHH (monomer) in the culture supernatant. The culture supernatant was collected by centrifugation at 3,000 xg, and bacterial cells were removed from the supernatant by treatment with a 0.22 µm filter.

### (5-3) Single point binding assay with Octet

Using Octet RED384 (manufactured by Fortebio Company), a His1K sensor chip was coated with the produced VHH clone to measure the activity to bind to each target molecule. A ligand was prepared such that the concentration of each target molecule was 400 nM, and 70 µL of a measurement liquid was added to a 384-well plate to measure the binding of each target molecule. Before the measurement, the tip of Dip and Read (trademark) His1K Biosensors (manufactured by Fortebio Company) was immersed in 200 µL of PBS-T (0.05% Tween 20, pH 7.4) for 10 minutes for hydration of the sensor chip. The condition of measurement order in each run measurement was as follows.

1) Baseline step: measured for 30 seconds in PBS-T
2) Loading step: measured for 100 seconds with VHH diluted 50-fold with PBS-T
3) Baseline step: measured for 30 seconds in PBS-T
4) Association step: measured for 100 seconds with each target molecule prepared in a form diluted with PBS-T
5) Dissociation step: measured for 100 seconds in PBS-T
6) Regeneration step: measured for 5 seconds in glycine-HCl (pH 2.2) and for 30 seconds in PBS-T

This step is repeated three times. The obtained data was processed using Octet Software Version (1.2.1.5) (Molecular Devices, LLC), and a clone having a Binding Response value of 0.1 or more was taken as a Hit clone.

### (5-3) Sequence analysis for Hit clone

For Hit clones, the VHH genes were identified by sequence analysis. A transformant taken as a Hit clone was cultured overnight at 37°C, and the culture solution was subjected to colony PCR. DNA purification was performed on the obtained PCR product using AMPure XP. Thereafter, analysis of each DNA was outsourced to Eurofins Genomics K.K. Among the obtained Hit clones, those identical in sequence were removed, and the remaining clones were taken as a Unique clone.

### (Example 6) In vitro analysis of characteristics of VHH clone

### (6-1) Acquisition of purified VHH

A Unique clone identified by the sequence analysis was pre-cultured overnight at 30°C. Thereafter, the preculture solution was passed to PM1S culture medium as a culture medium for VHH expression, and cultured at 25°C for 72 hours to secret and express VHH (monomer) in the culture supernatant. The culture supernatant was collected by centrifugation, and bacterial cells were removed from the supernatant.

Using His MultiTrap HP (manufactured by Cytiva Company), the above-described sample was purified from the obtained culture supernatant in accordance with the written instructions with the product. To this, 100 µL of an elution buffer (300 mM NaCl, 500 mM imidazole-containing 50 mM Tris-HCl (pH 7.5) was added, and centrifuged at 500 xg at 4°C for 2 minutes. The eluate was collected, and the eluate was used as a purified VHH clone sample.

### (6-2) Multipoint binding assay with Octet

Using Octet RED384, a His1K sensor chip was coated with the purified VHH clone in the sample, and the activity to bind to each target molecule was measured. In the measurement, seven concentrations of each target molecule in a 2-fold dilution series starting at 200 nM (200 nM, 100 nM, 50 nM, 25 nM, 12.5 nM, 6.25 nM and 3.125 nM) and a no target molecule condition were used, and 70 µL of each measurement solution was added to a 384-well plate. Before the measurement, the tip of Dip and Read (trademark) His1K Biosensors was immersed in 200 µL of PBS-T (0.05% Tween 20, pH 7.4) for 10 minutes, so that the sensor chip had affinity for water. The condition of measurement order in each run measurement was as follows.

1) Baseline step: measured for 30 seconds in PBS-T
2) Loading step: measured for 120 seconds with VHH diluted 50-fold with PBS-T
3) Baseline step: measured for 30 seconds in PBS-T
4) Association step: measured for 120 seconds with each target molecule prepared in a form diluted with PBS-T
5) Dissociation step: measured for 300 seconds in PBS-T
6) Regeneration step: measured for 5 seconds in glycine-HCl (pH 2.2) and for 30 seconds in PBS-T

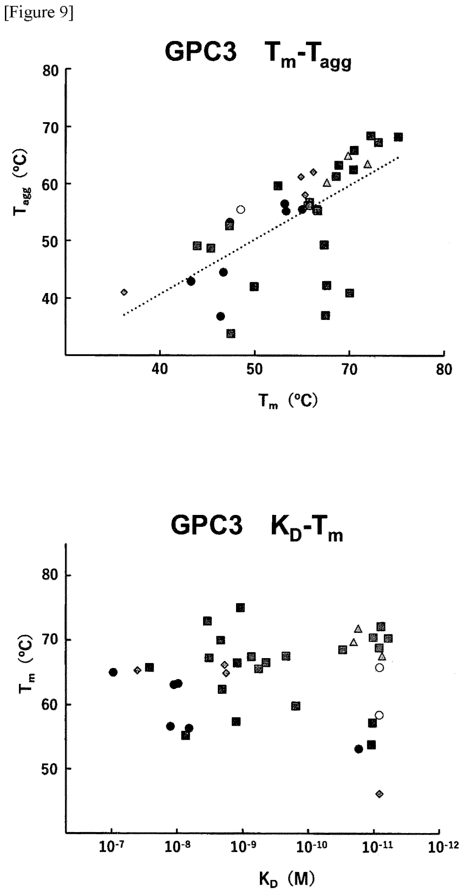

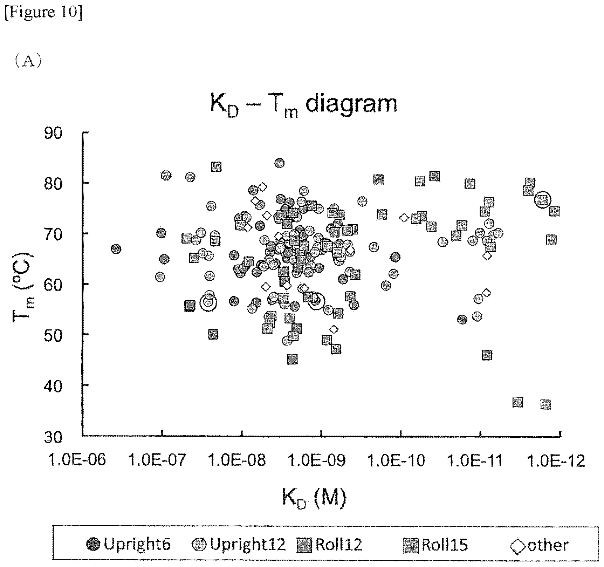

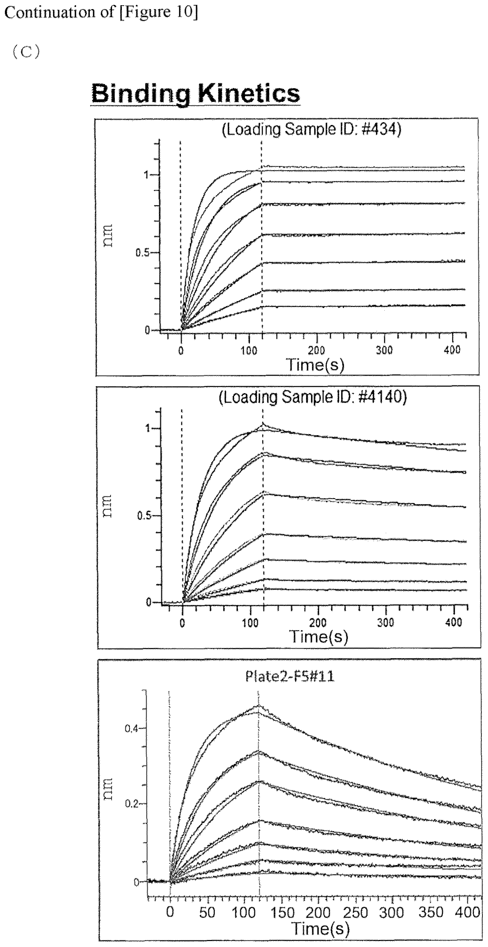

This step is repeated three times. Octet Software Version (1.2.1.5) was applied to the obtained analysis data to calculate the binding activity and the dissociation rate by taking a measurement at a target concentration of 0 nM as a reference and performing Global Fitting on the results of measurement under the conditions of 3.15 to 200 nM (Figures 9 and 10(A)).

### (6-3) Measurement of heat stability with UNcle

For the VHH clone purified as described above, a thermal denaturation midpoint (Tm value) and an aggregation initiation temperature (Tagg value) were derived using UNcle that is an apparatus for evaluating the physical properties of a protein.

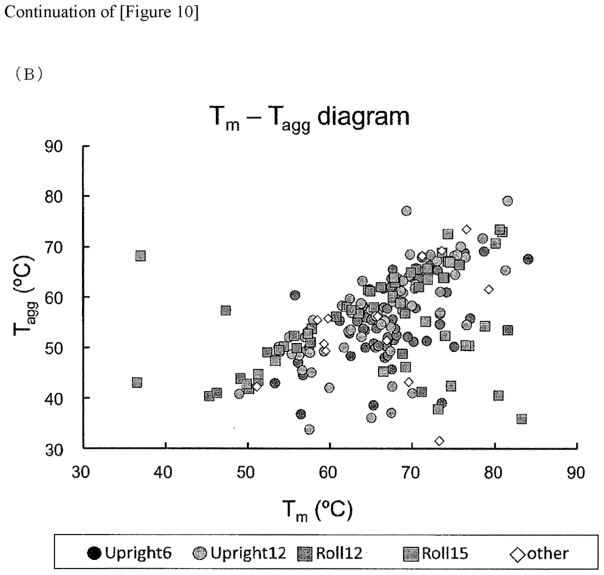

First, the purified VHH clone was applied to 16-well-9 µL quartz cuvettes, and the cuvettes were set in UNcle, followed by measurement. A change in fluorescence of the protein solution and scattered light was measured under measurement conditions conforming to the template set as a default in the equipment, where the initiation temperature was 25°C, the end-point temperature was 95°C, and the temperature was raised at 0.3°C/min. The thermal denaturation midpoint (Tm value) was calculated on the basis of a decrease in fluorescence intensity (due to heat denaturation of the protein) and a change in fluorescence spectrum over about 330 nm to 350 nm. An increase in average molecular weight of a solute (aggregation) by heat was measured by a change in intensity of scattered light at 266 nm, from which the aggregation initiation temperature (Tagg value) was calculated (Figures 9 and 10(B)).

### (Example 6) Evaluation of binding ability of VHH by flow cytometry

In Example 5, the characteristic of VHH were analyzed in vitro. However, the binding ability in vitro is not necessarily consistent with the binding ability in vivo. Thus, for more accurately evaluating the binding ability of VHH, the ability of VHH to bind to target proteins (GPC3 and HER2) expressed in cells was examined by flow cytometry (FCM).

In this experiment, hepG2 as hepatocyte cancer was selected as cells expressing GPC3, and SK-BR-3 as breast cancers was selected as cells expressing HER2. These cells were cultured to 80% confluence (in an incubator at 37°C and 5% CO₂, light shielded), and collected with PBS containing 5 mM EDTA. The collected cell sample was washed with 1% BSA/PBS, and a VHH solution diluted 100-fold with 1% BSA/PBS was added, followed by incubation at 4°C for 1 hour. The cells were washed three times with 1% BSA/PBS, and an AlexaFluor 488-bound anti-His tag antibody diluted 1000-fold (Medical & Biological Laboratory Co., Ltd.) was then added, followed by incubation at 4°C for 1 hour. The cells were washed with 1% BSA/PBS, and then resuspended with 1% BSA/PBS, and FCM analysis (SH800, Sony Corporation) was performed.

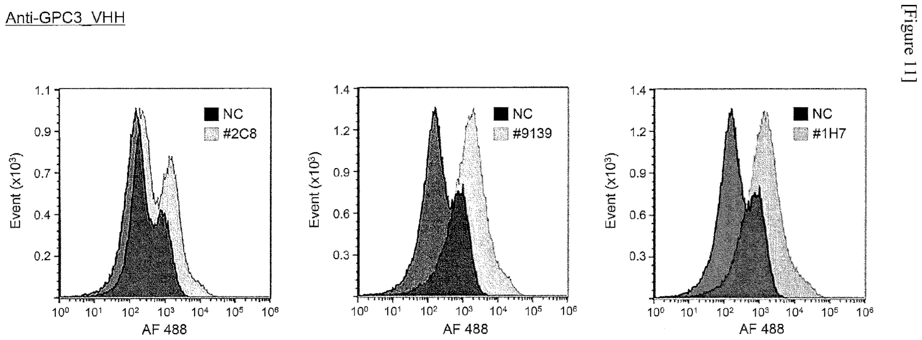

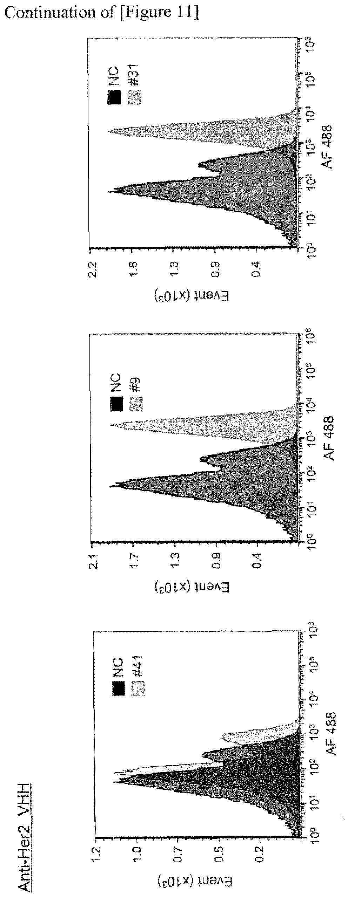

The results of the FCM analysis are shown in Figure 11. It has been shown that all VHHs shift to the left as compared to the negative control (NC), that is, bind specifically to cells expressing a target protein.

From the above, the DNA library having a framework according to the present invention has been confirmed to exhibit binding activity both in vitro and in vivo.

### Industrial Applicability

The invention of the present application is useful in the field of drug development.

### Sequence Listing Free Text

SEQ ID NO: 1 represents the amino acid sequence of FR1.
SEQ ID NO: 2 represents the amino acid sequence of FR2.
SEQ ID NO: 3 represents the amino acid sequence of FR2 (Upright type).
SEQ ID NO: 4 represents the amino acid sequence of FR2 (Upright type).
SEQ ID NO: 5 represents the amino acid sequence of FR2 (Roll type).
SEQ ID NO: 6 represents the amino acid sequence of FR2 (Roll type).
SEQ ID NO: 7 represents the amino acid sequence of FR3 (Upright type).
SEQ ID NO: 8 represents the amino acid sequence of FR3 (Upright type).
SEQ ID NO: 9 represents the amino acid sequence of FR3 (Upright type).
SEQ ID NO: 10 represents the amino acid sequence of FR3 (Upright type).
SEQ ID NO: 11 represents the amino acid sequence of FR3 (Upright type).
SEQ ID NO: 12 represents the amino acid sequence of FR3 (Upright type).
SEQ ID NO: 13 represents the amino acid sequence of FR3 (Upright type).
SEQ ID NO: 14 represents the amino acid sequence of FR3 (Upright type).
SEQ ID NO: 15 represents the amino acid sequence of FR3 (Upright type).
SEQ ID NO: 16 represents the amino acid sequence of FR3 (Upright type).
SEQ ID NO: 17 represents the amino acid sequence of FR3 (Upright type).
SEQ ID NO: 18 represents the amino acid sequence of FR3 (Upright type).
SEQ ID NO: 19 represents the amino acid sequence of FR3 (Upright type).
SEQ ID NO: 20 represents the amino acid sequence of FR3 (Upright type).
SEQ ID NO: 21 represents the amino acid sequence of FR3 (Upright type).
SEQ ID NO: 22 represents the amino acid sequence of FR3 (Upright type).
SEQ ID NO: 23 represents the amino acid sequence of FR3 (Upright type).
SEQ ID NO: 24 represents the amino acid sequence of FR3 (Upright type).
SEQ ID NO: 25 represents the amino acid sequence of FR3 (Upright type).
SEQ ID NO: 26 represents the amino acid sequence of FR3 (Upright type).
SEQ ID NO: 27 represents the amino acid sequence of FR3 (Upright type).
SEQ ID NO: 28 represents the amino acid sequence of FR3 (Upright type).
SEQ ID NO: 29 represents the amino acid sequence of FR3 (Upright type).
SEQ ID NO: 30 represents the amino acid sequence of FR3 (Roll/Upright type).
SEQ ID NO: 31 represents the amino acid sequence of FR3 (Roll/Upright type).
SEQ ID NO: 32 represents the amino acid sequence of FR4.
SEQ ID NO: 33 represents the nucleotide sequence of main chain of cnvK rC linker.
SEQ ID NO: 34 represents the amino acid sequence of FR1 of DP-47.
SEQ ID NO: 35 represents the amino acid sequence of FR2 of DP-47.
SEQ ID NO: 36 represents the amino acid sequence of FR3 of DP-47.
SEQ ID NO: 37 represents the amino acid sequence of FR4 of DP-47.

## Claims

1. A peptide consisting of three complementarity determining regions and four framework regions, and comprising an amino acid sequence represented by SEQ ID NO: 1 as a framework 1.

2. The peptide according to claim 1, wherein the framework region comprises an amino acid sequence represented by SEQ ID NO: 2 as a framework 2.

3. The peptide according to claim 2, wherein the framework 2 comprises an amino acid sequence selected from the group consisting of amino acid sequences represented by SEQ ID NOS: 3 to 6.

4. The peptide according to claim 1, wherein the framework region comprises an amino acid sequence represented by SEQ ID NO: 7 as framework 3.

5. The peptide according to claim 4, wherein the framework 3 comprises an amino acid sequence selected from the group consisting of amino acid sequences represented by SEQ ID NOS: 8 to 31.

6. The peptide according to claim 1, wherein the framework region comprises an amino acid sequence represented by SEQ ID NO: 32 as framework 4.

7. The peptide according to any one of claims 1 to 6, comprising a complementarity determining region 1 consisting of 10 amino acids, a complementarity determining region 2 consisting of 16 or 17 amino acids, and a complementarity determining region 3 consisting of 6, 12 or 15 amino acids.

8. A peptide library comprising the peptide according to claim 7.

9. A peptide library in which a peptide constituting the peptide library consists of three complementarity determining regions and four framework regions,
a framework 1 comprises an amino acid sequence represented by SEQ ID NO: 1,
a framework 2 comprises an amino acid sequence represented by any of SEQ ID NOS: 3 to 6,
a framework 3 comprises an amino acid sequence represented by any of SEQ ID NOS: 8 to 31,
a framework 4 comprises an amino acid sequence represented by SEQ ID NO: 32,
a complementarity determining region 1 is an amino acid sequence consisting of 10 amino acids,
a complementarity determining region 2 is an amino acid sequence consisting of 16 or 17 amino acids, and
a complementarity determining region 3 is an amino acid sequence consisting of 6, 12 or 15 amino acids.

10. The peptide according to claim 7, which is a single domain antibody derived from a heavy chain antibody.
